# EUROPEAN PATENT SPECIFICATION

(11) **EP 3 344 391 B1**
(45) Date of publication and mention of the grant of the patent: **15.03.2023**
(21) Application number: 16766759.1
(22) Date of filing: 02.09.2016
(51) Int. Cl.: B01L 3/00

(54) **DEVICES AND METHODS FOR MESOFLUIDIC AND/OR MICROFLUIDIC PROCESSES**
VORRICHTUNGEN UND VERFAHREN FÜR MESOFLUIDISCHE UND/ODER MIKROFLUIDISCHE VERFAHREN
DISPOSITIFS ET PROCÉDÉS POUR PROCESSUS MÉSOFLUIDIQUES ET/OU MICROFLUIDIQUES

(30) Priority: 04.09.2015 US 201562214630 P
(43) Date of publication of application: 11.07.2018
(73) Proprietor: Life Technologies Corporation, Carlsbad, CA 92008 (US)
(72) Inventor: LAFFERTY, William, Carlsbad, California 92008 (US); BASHTANOV, Mikhail, Cambridge Cambridgeshire CB3 0QE (GB); CROSSLEY, Peter, Cambridge Cambridgeshire CB23 7EQ (GB); GRAHAM, Abi, Cambridge Cambridgeshire CB1 3QN (GB); JEPPS, Gary, Cambridge Cambridgeshire CB22 5BX (GB); LINTERN, Richard, Cambridge Cambridgeshire CB24 9LI (GB); LOWE, Stuart, Bishop's Stortford Hertfordshire CM23 3SL (GB); LU, Yang, Cambridge Cambridgeshire CB13HP (GB); ONSLOW, James, Huntingdon Cambridgeshire PE28 5TX (GB); POLLOCK, Neil, Wimpole Hertfordshire SG8 5QD (GB); RAMMENSEE, Sebastian, Upminster RM14 3LD (GB); JANSE VAN RENSBURG, Richard, Great Cambourne Cambridgeshire CB23 6GP (GB); WAKEFIELD, Steve, Baldock Hertfordshire SG7 6DB (GB); EUGSTER, Peter, San Mateo CA 94403 (US)
(74) Representative: Hoffmann Eitle
(86) International application number: PCT/US2016/050246
(87) International publication number: WO 2017/041023

(56) References cited:
- WO-A1-03/049860
- WO-A1-2011/151804
- WO-A2-2005/121963
- WO-A2-2005/121963
- WO-A2-2014/022700
- DE-A1-102005 049 976
- US-A- 5 846 446
- US-A- 5 846 446
- US-A- 5 846 446
- US-A1- 2003 049 833
- US-A1- 2003 049 833
- US-A1- 2004 121 471
- US-A1- 2008 187 469
- US-A1- 2009 023 201

## Description

### BACKGROUND

The present disclosure relates generally to devices and methods for mesofluidic and/or microfluidic processes, such as polymerase chain reactions and/or DNA sequencing. More specifically, this disclosure relates to devices and methods for mesofluidics and/or microfluidics to perform biomolecular processes, assays, and workflows, such as: nucleic acid extraction; PCR; preparation of biological samples for isolation, separation, and detection of biomolecules including nucleic acids and proteins; DNA sequencing; qPCR; immunoassays; preparation and genetic manipulation of cells; and immunotherapy.

The polymerase chain reaction (PCR) identifies and replicates strands of deoxyribonucleic acid (DNA) and is employed in modern techniques of genetic analysis. The principle of PCR is in vitro multiplication of strands of DNA using enzymatic polymerases.

The duplication of a DNA strand in PCR is carried out in three principle steps. First, the original double- stranded DNA is split in two single- strands of DNA, a process known as "denaturation" or "melting." Second, primers attach to defined sites of the single-strands of DNA. Third, starting from the primer, a polymerase attaches to the single-strand DNA and forms a complementary DNA strand, thereby forming an identical (or almost identical) copy of the original double- stranded DNA. The process is then repeated multiple times to achieve an exponential growth of the number of identical DNA strands. Analysis of the resultant pool of DNA can then be performed.

PCR is classically performed in a device called a thermal cycler. Specifically, a small plastic tube contains the DNA sample together with the primer and polymerase molecules in a suitable buffer, and the thermal cycler repeatedly temperature cycles the tube through the PCR phases described above, for example between 15 and 25 times in typical applications.

WO 2005/121963 A2 describes a sample processing cartridge including a plurality of segments arranged in an array at least two rows long and two columns wide. US 5 846 446 describes an ice making bag comprising an envelope which comprises an envelope top and an envelope bottom. The ice making bag further comprises a binding which comprises a peripheral binding.
US 2003/049833 A1 describes a sample vessel comprising a tubule having an opening for receiving a sample material and at least one compressible section, a generally rigid container receiving at least a portion of the tubule; and an interface in fluid communication with the opening in the tubule.

### SUMMARY

The present invention relates to an automated system of claim 1 and a method of claim 4. Prefered embodiments are set out in the dependent claims.

The present inventors recognized several problems with known methods and devices for mesofluidic and/or microfluidic processes such as continuous-flow PCR and DNA sequencing. One or more of these problems can be addressed by devices in a plurality of stages of fluid handling and processing which are automated, using an instrument and a complimentary customized disposable cartridge. The cartridge includes one or more integral flow paths that allow fluids or reagents to pass from one processing stage to the next without need for manual user handling or risk of contamination by employing a multitude of different laboratory techniques. The instrument includes several mechanical components that act on different portions of the disposable cartridge to accomplish the processing stages desired by the user.

One such disposable cartridge includes portions of a first elastomeric membrane that abut or are proximate to portions of a second elastomeric membrane, and these portions of the first and second elastomeric membranes can be sequentially pushed apart by a fluid to form a channel for the fluid with minimal or no dead volume. Preferably these channel-forming portions of the first and second elastomeric membranes are circumscribed by sealed portions of the first elastomeric membrane that are fixedly attached to corresponding sealed portions of the second elastomeric membrane.

Biomolecular assays and workflows can require complex manipulations of samples and reagents. A user benefits from simplifying such complex manipulations by automation and integration. Automating and integrating makes the processes more efficient, more robust, more easily repeatable, simpler to use, and more cost effective.

Therefore, the present disclosure provides a system which integrates the following functionality to enable a vast array of potential workflows and assays, including but not limited to, for example: pumping and valving workflows; integrated storage and release of wet and dry reagents across a broad range of volumes; precise thermal control of reagents and samples; solid phase isolation of reactants, intermediates, reaction products, and biomolocules via magnetic, paramagnetic, non-magnetic beads and coated films or substrates; and optical tools to detect reactions and intermediates. Finally, such an automated system has the ability to link each of the above exemplary workflows and associated functionality together. Linking functionality together as combinatorial building blocks enables rapid deployment of workflows that span from simple add and mix to complex fully integrates sample-to-answer systems that include processing samples through multiple sequential rounds of each of the above building blocks.

Additional features are described herein and will be apparent from the following Detailed Description and the Figures.

### BRIEF DESCRIPTION OF THE FIGURES

**FIG. 1** depicts a cross-sectional view of one example of the disclosure.
**FIG. 2A** depicts a cross-sectional view of a groove of one example of the disclosure.
**FIG. 2B** depicts a cross-sectional view of a groove of one example of the disclosure.
**FIG. 2C** depicts a cross-sectional view of a groove of one example of the disclosure.
**FIG. 3A** illustrates an isometric view of one example of the disclosure.
**FIG. 3B** illustrates a bottom view of one example of the disclosure.
**FIG. 4A** illustrates an isometric view of one example of the disclosure.
**FIG. 4B** illustrates a bottom view of one example of the disclosure.
**FIG. 5** depicts different views of the edge of a groove of one embodiment of the disclosure.
**FIG. 6** depicts a cross-sectional view of one example of the disclosure.
**FIG. 7** illustrates a picture of a thermal plastic sheet sealing in an embodiment of the disclosure.
**FIG. 8** depicts a cross-sectional view of one embodiment of the disclosure.
**FIG. 9A** depicts a cross-sectional view of one embodiment of the disclosure.
**FIG. 9B** depicts a cross-sectional view of a groove of one embodiment of the disclosure.
**FIG. 9C** depicts a top view of chambers of one embodiment the disclosure.
**FIG. 9D** depicts a cross-sectional view of a lane assembly of one embodiment of the disclosure.
**FIG. 10A** depicts a cross-sectional view of one embodiment of the disclosure.
**FIG. 10B** depicts a cross-sectional view of a groove of one embodiment of the disclosure.
**FIG. 10C** depicts a top view of chambers of one embodiment the disclosure.
**FIG. 10D** depicts a cross-sectional view of a lane assembly of one embodiment of the disclosure.
**FIG. 11A** depicts a cross-sectional view of one embodiment of the disclosure.
**FIG. 11B** depicts a cross-sectional view of a groove of one embodiment of the disclosure.
**FIG. 11C** depicts a top view of chambers of one embodiment the disclosure.
**FIG. 12A** depicts a cross-sectional view of one embodiment of the disclosure.
**FIG. 12B** depicts a cross-sectional view of a groove of one embodiment of the disclosure.
**FIG. 12C** depicts a top view of chambers of one embodiment the disclosure.
**FIG. 13** depicts a cross-sectional view of one embodiment of the disclosure.
**FIG. 14** depicts a cross-sectional view of a flow path of one embodiment of the disclosure.
**FIG. 15** depicts a cross-sectional view a flow path of one embodiment of the disclosure.
**FIG. 16** illustrates an isometric exploded view of an assembly of one embodiment of the disclosure.
**FIG. 17** illustrates an isometric exploded view of an assembly of one embodiment of the disclosure.
**FIG. 18A** illustrates a cross-sectional exploded view of an assembly of one embodiment of the disclosure.
**FIG. 18B** illustrates a cross-sectional assembled view of one embodiment of the disclosure.
**FIG. 19** illustrates a cross-sectional view of one embodiment of the disclosure.
**FIG. 20A** illustrates a cross-sectional view of the membrane fluid path of one embodiment of the disclosure.
**FIG. 20B** illustrates a cross-sectional view of one embodiment of the disclosure.
**FIG. 21** illustrates a cross-sectional view of a flow path of one embodiment of the disclosure.
**FIG. 22A** illustrates a photograph of a heater block of one embodiment of the disclosure.
**FIG. 22B** illustrates a representation of a heater block of one embodiment of the disclosure.
**FIG. 22C** illustrates a representation of a heater block of one embodiment of the disclosure.
**FIG. 23A** illustrates a cross-sectional view of one embodiment of the disclosure.
**FIG. 23B** illustrates a cross-sectional view of one embodiment of the disclosure.
**FIG. 23C** illustrates a cross-sectional view of one embodiment of the disclosure.
**FIG. 24A** illustrates a cross-sectional view of one embodiment of the disclosure.
**FIG. 24B** illustrates a cross-sectional view of one embodiment of the disclosure.
**FIG. 25A** illustrates a side view of the elastomeric membranes of one embodiment of the disclosure.
**FIG. 25B** illustrates a side view of the elastomeric membranes of one embodiment of the disclosure.
**FIG. 26A** illustrates a photograph of a mixing chamber of the membranes of one embodiment of the disclosure.
**FIG. 26B** illustrates a photograph of a mixing chamber of the membranes of one embodiment of the disclosure.
**FIG. 26C** illustrates a photograph of a mixing chamber of the membranes of one embodiment of the disclosure.
**FIG. 26D** illustrates a photograph of a mixing chamber of the membranes of one embodiment of the disclosure.
**FIG. 27A** illustrates a cross-section of an elastomeric membrane of one embodiment of the disclosure.
**FIG. 27B** illustrates a cross-section of an elastomeric membrane of one embodiment of the disclosure.
**FIG. 27C** illustrates a cross-section of an elastomeric membrane of one embodiment of the disclosure.
**FIG. 28A** illustrates a cross-section of an elastomeric membrane of one embodiment of the disclosure.
**FIG. 28B** illustrates a cross-section of an elastomeric membrane of one embodiment of the disclosure.
**FIG. 29** depicts the flow distribution through various mixing chamber geometry embodiments of the disclosure.
**FIG. 30A** illustrates a cross-section of a flow path of one embodiment of the disclosure.
**FIG. 30B** illustrates a cross-section of a flow path of one embodiment of the disclosure.
**FIG. 30C** depicts a schematic of a flow path of one embodiment of the disclosure.
**FIG. 30D** depicts a schematic of a flow path of one embodiment of the disclosure.
**FIG. 30E** depicts a schematic of a mixing chamber with mixing beads of one example of the disclosure, not forming part of the present invention.
**FIG. 31** illustrates a photograph of a mixing chamber embodiment of the disclosure.
**FIG. 32A** illustrates a view of elastomeric membranes of one embodiment of the disclosure.
**FIG. 32B** illustrates a view of elastomeric membranes having elastomeric patches of one embodiment of the disclosure.
**FIG. 32C** illustrates a cross-sectional view of one embodiment of the disclosure.
**FIG. 33A** illustrates a photograph of one embodiment of the disclosure.
**FIG. 33B** illustrates a photograph of one embodiment of the disclosure.
**FIG. 34A** illustrates a photograph of a cross-sectional view of one embodiment of the disclosure.
**FIG. 34B** illustrates a photograph of one embodiment of the disclosure.
**FIG. 35** illustrates a photograph of one embodiment of the disclosure.
**FIG. 36** illustrates a series of photographs showing fluid moving through one embodiment of the disclosure.
**FIG. 37** illustrates a photograph of reagents flowing through the elastomeric membranes of one embodiment of the disclosure.
**FIG. 38** illustrates an isometric view of an exploded assembly of one embodiment of the disclosure.
**FIG. 39** illustrates a view of a partial assembly of one embodiment of the disclosure.
**FIG. 40** illustrates a view of a partial assembly of one embodiment of the disclosure.
**FIG. 41** illustrates a schematic of a partial assembly of one embodiment of the disclosure.
**FIG. 42** illustrates a view of a bubble trap embodiment of the disclosure
**FIG. 43A** illustrates a photograph of one embodiment of the disclosure.
**FIG. 43B** illustrates a photograph of one embodiment of the disclosure.
**FIG. 44** illustrates a volumetric schematic of one embodiment of the disclosure.
**FIG. 45** illustrates a side cross-section exploded view of twin blisters in one embodiment of the disclosure.
**FIG. 46** illustrates a side cross-section view of manufacture of twin blisters in one embodiment of the disclosure.
**FIG. 47** illustrates a side cross-section view of use of twin blisters in one embodiment of the disclosure.
**FIG. 48** illustrates a side cross-section view of use of twin blisters in one embodiment of the disclosure.
**FIG. 49A** is a flowchart of workflow in known preparation and processing methods.
**FIG. 49B** is a flowchart of workflow in one embodiment of the disclosure.
**FIG. 50** illustrates an instrument with cartridges in one embodiment of the disclosure.

### DETAILED DESCRIPTION

As used in this disclosure and the appended claims, the singular forms "a," "an" and "the" include plural referents unless the context clearly dictates otherwise. Thus, for example, reference to "a material" or "the material" includes two or more materials. The term "and/or" used in the context of "X and/or Y" should be interpreted as "X," or "Y," or "X and Y." Where used herein, the term "example," particularly when followed by a listing of terms, is merely exemplary and illustrative, and should not be deemed to be exclusive or comprehensive.

As used herein, "about," "approximately" and "substantially" are understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. "Similarly dimensioned" means that the referenced components have at least two dimensions that are substantially the same as the corresponding dimensions of the other components, and in some embodiments three of such dimensions.

All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

Numerical adjectives, such as "first" and "second," are merely used to distinguish components. These numerical adjectives do not imply the presence of other components, a relative positioning, or any chronological implementation. In this regard, the presence of a "second opening" does not imply that a "first opening" is necessarily present. Further in this regard, a "second opening" can be upstream from, downstream from or co-located with a "first opening," if any; and a "second opening" can be used before, after, and/or simultaneously with a "first opening," if any.

The devices disclosed herein may lack any element that is not specifically disclosed herein. Thus, a disclosure of an embodiment using the term "comprising" includes a disclosure of embodiments "consisting essentially of" and "consisting of" the components identified. Similarly, the methods disclosed herein may lack any step that is not specifically disclosed herein. Thus, a disclosure of an embodiment using the term "comprising" includes a disclosure of embodiments "consisting essentially of" and "consisting of" the steps identified.

Various embodiments of a mesofluidic and/or microfluidic device are disclosed herein, and any embodiment can be combined with any other embodiment unless explicitly and directly stated otherwise. In some embodiments, the device is a cartridge configured for a continuous-flow process that is PCR, DNA sequencing, or a combination thereof. As used herein, "continuous-flow" means that the initial sample is added to the cartridge and undergoes the process to completion without the need to be transferred from the cartridge to a different device.

### Known Sample Prep Methods and Designs

Known systems for sample prep, PCR, and DNA sequencing typically involve several independent modules or instruments depending upon the particular stage of the process. In most known systems, each individual module or step of the process requires a separate instrument acting on the fluid, which therefore necessitates multiple phases of manual instrument set-up and handling of the fluid by an operator. For example, in one instance of sequencing prep, the typical known methods include a combination of lab bench workflow and instrument use.

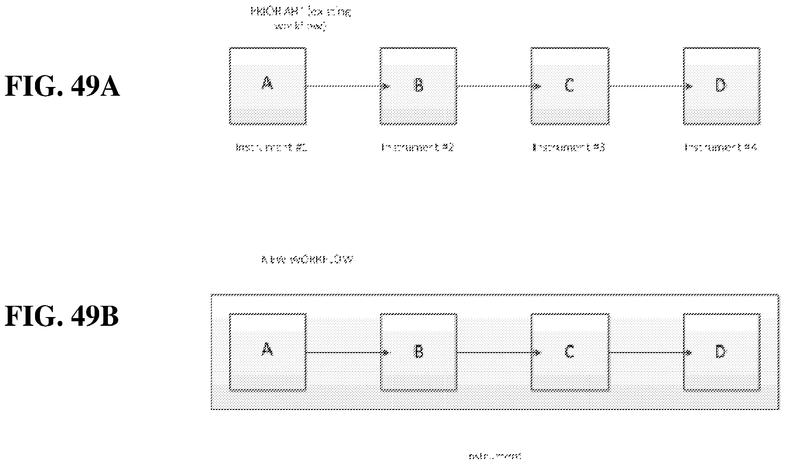

**FIG. 49A** is a schematic flow-chart representative of known methods for sample preparation and processing. The workflow includes four stages: A, B, C and D, each of which requires the use of a separate respective instrument: Instrument #1, #2, #3 and #4. In one example embodiment, Stage A requires DNA extraction from a raw sample. The Instrument #1 to accomplish such extraction includes largely manual work including pipettes, tubes/plates, a vortexter, a centrifuge, and a magnet rack for bead handling. Following the DNA extraction of Stage A, the sample must undergo thermal cycling in Stage B, using Instrument #2, which would typically be a commercially available thermal cycler. Stage C also includes the use of a thermal cycler, but requires the user to add reagents to the plate after the thermal cycling of Stage B and before thermal cycling of Stage C begins. Finally, Stage D can be a sequencing clean-up workflow, which is typically accomplished manually. Instrument #4 used in Stage D can involve many of the same tools as Instrument #1 in Stage A, such as pipettes, a centrifuge, a vortexter, and a magnetic rack.

It should be appreciated that the manual workflows described in Stages A and D, as well as the manual reagent supplementation required between Stages B and C, are accompanied by a plurality of inefficiencies and variables. A process such as that described above and illustrated in **FIG. 49A** requires very precise and consistent user execution that must be done efficiently, reliably, and without damaging or contaminating the sample during the process.

### Overview of Automated Sample Prep Methods and Devices

In various embodiments provided by the present disclosure, each of Stages A to D, as well as all the Instruments required to execute Stages A to D, are integrated into a single automated system that includes an instrument and a complimentary corresponding cartridge. As depicted in **FIG. 49B****,** the new workflow disclosed herein encapsulates each of Stages A, B, C, and D within a single instrument, thus eliminating the risks of error, inconsistency, or contamination of the sample, and therefore decreasing cost of producing repeatable samples.

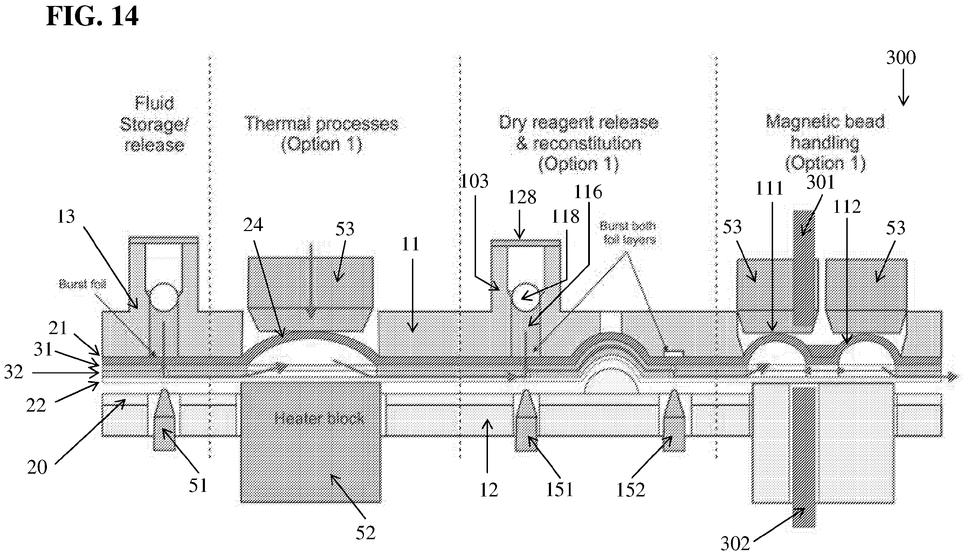

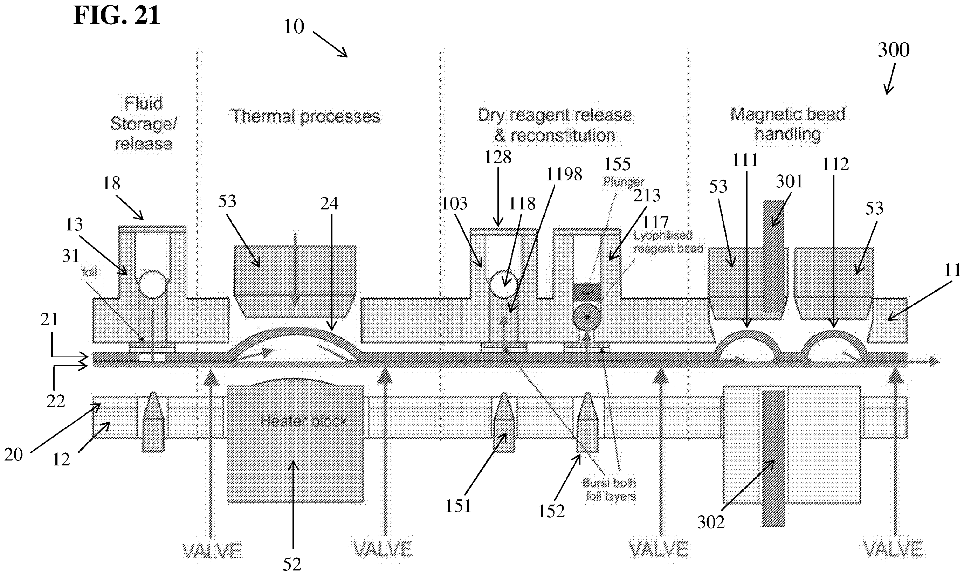

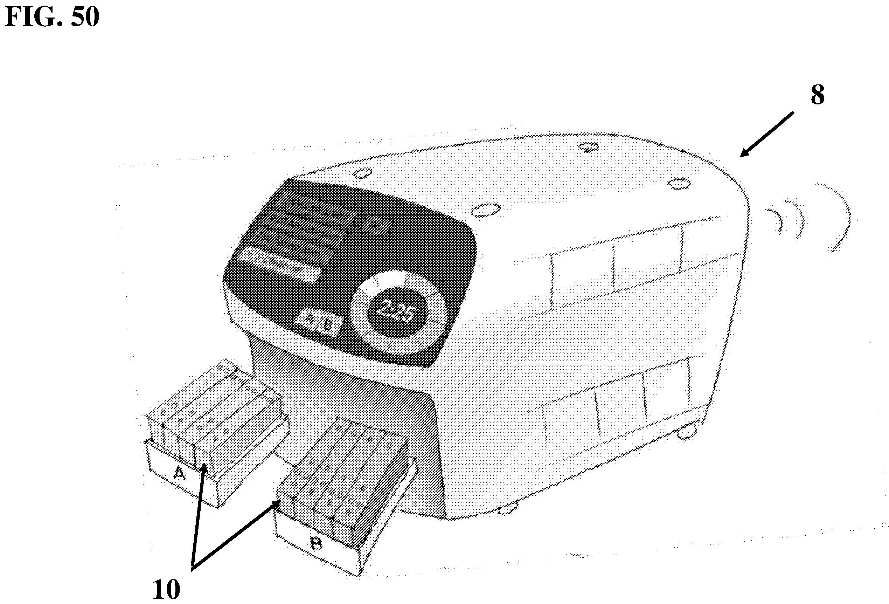

In various embodiments of the present disclosure, as generally illustrated in **FIGS. 14****,** **21****,** and **50****,** a device for processes such as sample preparation, PCR, and DNA sequencing includes an integrated flow path with a plurality of different modules for processing the subject fluid. In various embodiments discussed herein and generally illustrated in **FIG. 50****,** such a device includes an instrument 8 with a plurality of mechanical components and a complementary disposable cartridge 10 configured to contain any fluid or reagents to be processed. Exemplary flow paths and processes of a disposable cartridge are illustrated in detail in **FIGS. 14** and **21****.** In various embodiments, the mechanical components of the instrument 8 are configured to act upon appropriate locations of the cartridge 10 to enable fluid flow in a fluid flow path, extract DNA from a raw sample, provide thermal cycling, introduce and mix reagents, perform sequencing clean-up, as well as other tasks while minimizing user handling in between stages of the process. By customizing the cartridge 10 to include the selected reagents, processes, fluid pathways, and specifications of a user's defined goal, the instrument 8 can be programmed to automate the entire workflow without requiring individual manual instrument set-up and unnecessary fluid handling by the user.

It should be appreciated that an automated system, as illustrated generally in **FIGS. 14****,** **21** and **50** and discussed throughout, would be more cost effective than known multi-instrument systems by providing greater sample quality, repeatability, and predictability. Further, enabling customization of the cartridge 10 to be used in a common instrument gives the user increased flexibility not available in current systems. The cartridge 10 can perform at least equivalently to the manual lab techniques discussed above and shown in **FIG. 49A****.**

Another advantage of one or more embodiments of the system provided by the present disclosure is that the disposable cartridge 10 has all necessary reagents on-board. The cartridge 10 can enable multiplex, concurrent testing of multiple samples, for example 8-12 samples. Moreover, the cartridge 10 can comprise multiple individual pathways, each of which can process a single sample. The cartridge 10 can be configured by the user to process a desired number of samples. Notably, the cartridge 10 is not limited to one specific type of processing, and the cartridge 10 can be easily configured for any type of mesofluidic and/or microfluidic processing.

Preferably the cartridge 10 cooperatively connects to the instrument 8 to avoid the need for accurate alignment between the instrument 8 and the lanes of the cartridge 10. In an embodiment, pneumatic connection of the cartridge 10 to the instrument 8 is not required. Eliminating pneumatic connection avoids the risk of cross-contamination and aerosolizing fluids, the poor reliability of air connections, and the need for calibration of the air source (pressure). Generally, the instrument 8 in which the cartridge is used 10 is simplified relative to known mesofluidic and/or microfluidic devices, to maximize reliability and to reduce cost.

In an embodiment generally illustrated in **FIG. 14****,** partial cross sectional views of both the cartridge 10 and the instrument 8 are displayed. The cartridge 10, for example, can include at least a first chassis 11; a support block 12 having a compliant layer 20; first and second elastomeric membranes 21, 22; and first and second foil layers 31, 32. In various embodiments, the support block 12 can form one or more valves in the cartridge 10. The mechanical elements of the instrument 8 can include at least plungers 51, 151 and 152, a heater block 52, pistons 53, and upper and lower magnets 301 and 302 respectively.

As seen in **FIG. 14****,** four example stages are illustrated and each stage is separated by vertical dashed lines. In the illustrated embodiment, the first stage is the fluid storage/release, wherein the initial fluid is forced into the flow path of the cartridge as discussed in greater detail below. As the flow continues toward the right of the illustration, the fluid undergoes thermal processing in the second stage. During thermal processing, the permanent heater block or heater blocks that are disposed within the instrument act upon the fluid flow path of the disposable cartridge to enable seamless transition from the first fluid storage/release stage to the thermal processing stage. The third exemplary stage illustrated in **FIG. 14** is the addition and reconstitution of a dry reagent. As seen in **FIG. 14****,** the dry reagent release and reconstitution is enabled by the bursting of foil layers on the cartridge using plungers 151, 152, which are permanent mechanical components integrated into the instrument 8.

Finally, the fourth exemplary stage illustrated in **FIG. 14** is the magnetic bead handling of the sample. The magnetic bead handling mechanism 300 can be configured to bind, wash and elute the amplified DNA. The magnetic bead handling mechanism 300 can comprise one or more magnets, for example the upper magnet 301 and/or the lower magnet 302. Part of the instrument in various embodiments, the magnets 301, 302 can be motor-driven. Each of the stages illustrated in **FIG. 14** is discussed in greater detail below.

**FIG. 21** illustrates a similar configuration of an overview of the automated process as the arrangement discussed above and illustrated in **FIG. 14****.** Among other distinctions, **FIG. 21** includes a lyophilized reagent bead pot 213 having a plunger 155 for introduction of a lyophilized reagent bead into the flow path between membranes 21 and 22. The reagent within pot 213 is put into fluid communication with the flow path defined between elastomeric membranes 21 and 22, preferably fluid communication through a foil layer or layers which are burst by the plunger 152 which can be provided by the instrument 8. Additional variations and discussion of the embodiment depicted in **FIG. 21** are discussed in greater detail later herein.

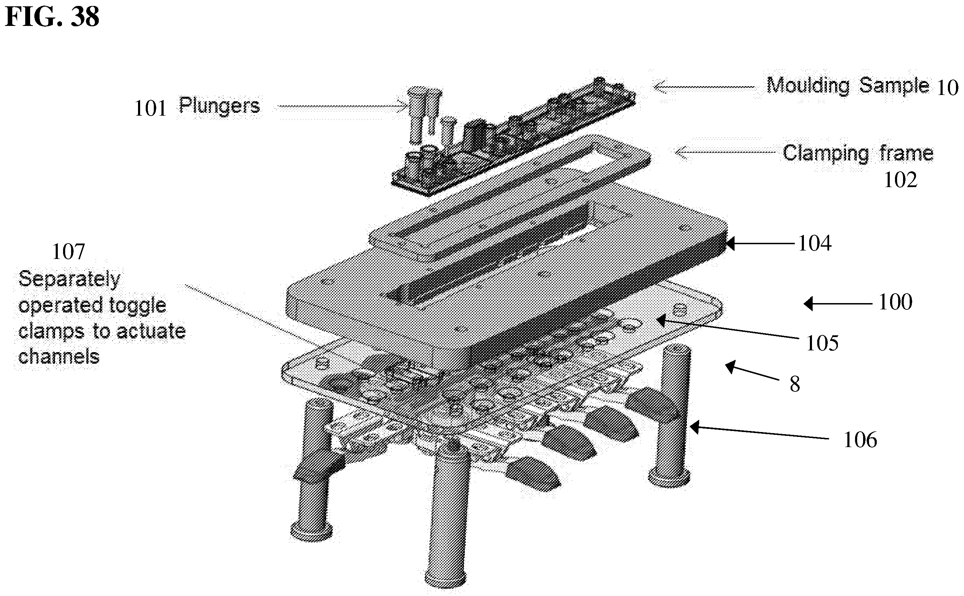

Referring now to **FIG. 38****,** an exploded view of a portion of the manual system is illustrated and described, however one skilled in the art would recognize that such a system can also be automated. One embodiment of cartridge 10 is illustrated with its several pots and a plurality of associated plungers to be disposed in each of said pot. The cartridge 10 is situated on top of a suitable clamping frame. It should be appreciated that in several embodiments, one or more of the clamping frame, the instrument chassis, and all of the individual clamps and plungers on the underside thereof are permanent parts of the instrument 8. The layout on the cartridge 10 of the flow path(s), the pot(s) for reagent and/or fluid introduction, the mixing chamber(s), the heater blocks for thermal cycling and PCR, the magnetic bead handling mechanisms and sample cleanup can be designed in many contemplated configurations due to the plurality of toggle clamps, plungers and other permanent mechanisms of the instrument 8. It should be appreciated that, in various embodiments discussed below, the toggle clamps of the instrument 8 act as valve actuators, which impart pressure upon different portions of the cartridge to define or obstruct flow paths, and to aid in pumping and directing fluid flow from initial introduction of the sample, through sample prep, and final clean-up.

As can be seen from **FIGS. 14****,** **21** and **38****,** the system comprising the instrument 8 and the cartridge 10 simplifies the interfaces between the cartridge 10 and the instrument 8 in which the cartridge 10 is used (e.g. minimizes or avoids fluid/gas connections, avoids complex mechanical attachments to the lanes of the cartridge 10, etc.). Moreover, the number of types of each interface is minimized (e.g. one type of mechanical interface).

### Cartridge Construction and Design

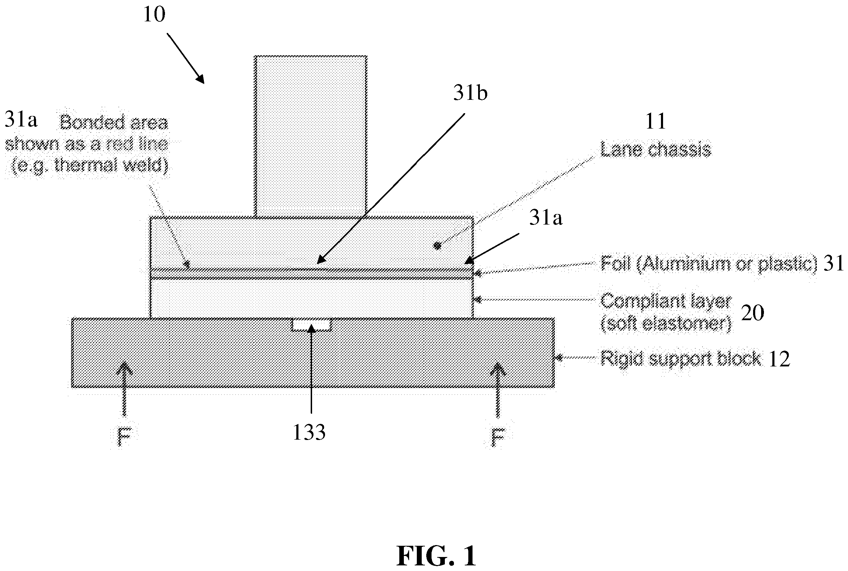

In an example generally illustrated in **FIGS. 1****, 2A-2C****,** **3A****,** **3B****,** **4A** and **4B,** a detailed view of one example of the cartridge 10 is disclosed. The cartridge 10 can comprise a first chassis 11. The first chassis 11 can be formed at least partially by a polypropylene material, a cyclic olefin copolymer (COC), or any other suitable material or combination thereof. The first chassis 11 can form at least part of a frame of the cartridge 10.

A first foil layer 31 can be positioned under the first chassis 11. At least a portion of the upper surface of the first foil layer 31 can abut the first chassis 11, preferably the entirety of the upper surface of the first foil layer 31. The upper surface of the first foil layer 31 can comprise sealed portions 31a that are thermally welded, laser-welded, bonded by pressure-sensitive adhesive, or otherwise fixedly attached to the first chassis 11. The first foil layer 31 can comprise aluminum or a plastic, such as polypropylene (PP), and can be smooth or corrugated. As a non-limiting example, the first foil layer 31 can have a thickness of about 37 µm.

A compliant layer 20 can be positioned under the first foil layer 31. The compliant layer 20 can reversibly move between a closed position abutting the first foil layer 31 and an open position out of contact from the first foil layer 31, for example, by moving a support block 12 to which the compliant layer 20 can be fixedly attached. In a preferred embodiment, the instrument 8 in which the cartridge 10 is used provides the support block 12 and the compliant layer 20.

The upper surface of the first foil layer 31 comprises unsealed portions 31b circumscribed by the sealed portions 31a. The sealed portions 31a of the first foil layer 31 can thus define a periphery of the unsealed portions 31b of the first foil layer 31 such that the distance between the sealed portions 31a in any particular direction is the width of the unsealed portions 31b in the same direction. The unsealed portions 31b can extend approximately the entire length of the cartridge 10, continuous with each other and uninterrupted by the sealed portions 31a, such that the unsealed portions 31b are configured to form a channel, as discussed in greater detail hereafter. As a non-limiting example, the channel may have a length of about 10 mm.

Fluid, for example fluid containing one or more reagents for PCR and/or DNA sequencing, can be positioned between the first chassis 11 and a section of the unsealed portions 31b of the first foil layer 31. Advantageously, the cartridge 10 can enable an initial sample type that is a pipetted liquid. The fluid can be urged onto an adjacent downstream unsealed portion 31b of the first foil layer 31, for example by positive displacement and preferably without pneumatic displacement, thereby pushing the downstream unsealed portion 31b of the first foil layer 31 away from the first chassis 11. Consequently, the unsealed portion 31b of the first foil layer 31 can be sequentially pushed away from the first chassis 11 to form a channel between the first foil layer 31 and the first chassis 11.

The sealed portions 31a of the first foil layer 31 can thus define a periphery of the channel formed between the first chassis 11 and the unsealed portions 31b of the first foil layer 31; for example, if the sealed portions 31a of the first foil layer 31 are about 2 mm apart, the channel can have a width of about 2 mm.

As the fluid progresses downstream, the upstream unsealed portions 31b of the channel which the fluid has evacuated can return to the resting state in which the first foil layer 31 is substantially flat. In a preferred embodiment, the compliant layer 20 is pushed into the unsealed portions 31b which contain the fluid to positively displace the fluid downstream and close the channel behind the fluid, for example by pushing the unsealed portions 31b containing the fluid to be substantially flat. If the upstream unsealed portions 31b are also held substantially flat by the compliant layer 20, the fluid can travel downstream. The pressure required to close the channel can depend on the stiffness and thickness of the compliant layer 20, the geometry of the compliant layer 20, and the channel dimensions.

A groove 133 can facilitate formation of the channel, for example by allowing the unsealed portions 31b of the first foil layer 31 to expand. Preferably the width of the groove 133 should be about equal to the width of the unsealed portions 132 of the first foil layer 31 and thus about equal to the width of the resultant channel. As a non-limiting example, the groove 133 can have a width of about 2 mm to about 3 mm and a depth of at least about 20 µm, for example about 50 µm to about 100 µm.

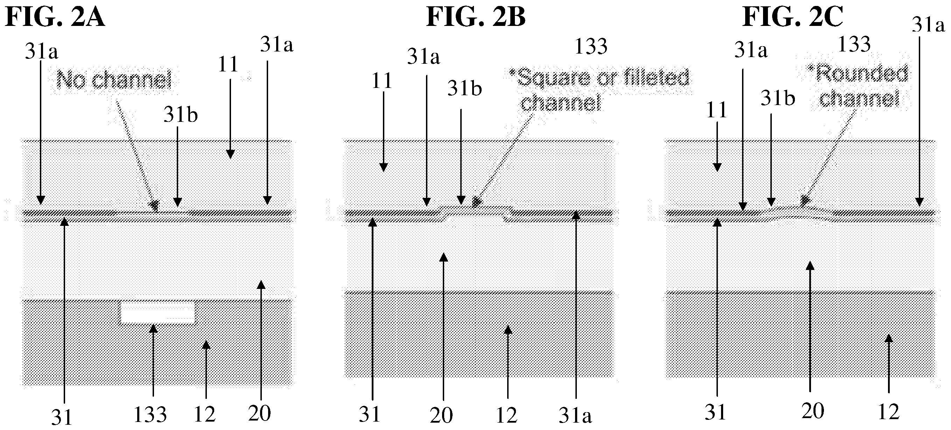

In an example depicted in **FIG. 2A****,** the support block 12 can comprise the groove 133. In an embodiment depicted in **FIGS. 2B** and **2C****,** the first chassis 11 can comprise the groove 133, and the groove 133 can form an opposite side of the channel from the first foil layer 31. In **FIG. 2B****,** the groove 133 is square or filleted, and in **FIG. 2C** the groove 133 is rounded. In these examples, pressure applied through the compliant layer 20 can force the unsealed portions 132 of the first foil layer 31 to conform to the groove 133 and thereby close the channel.

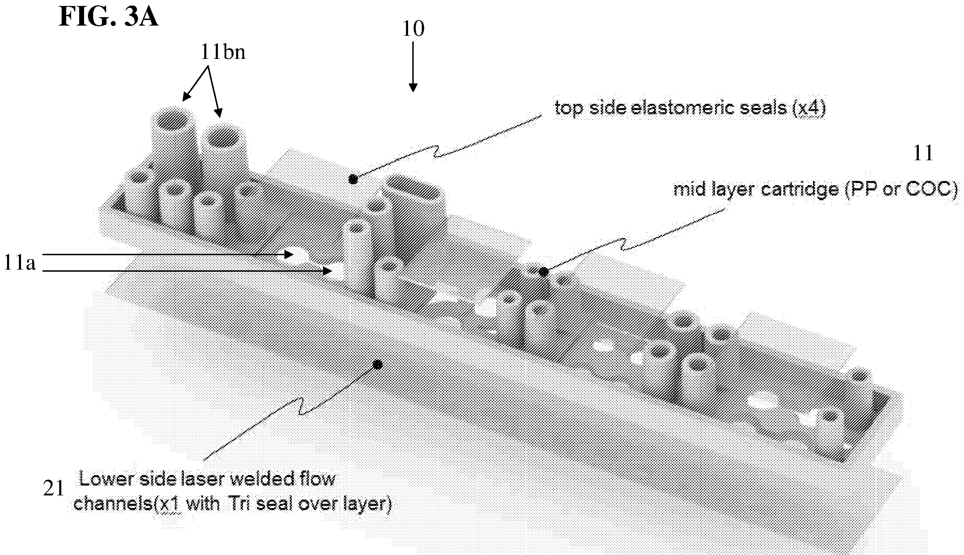

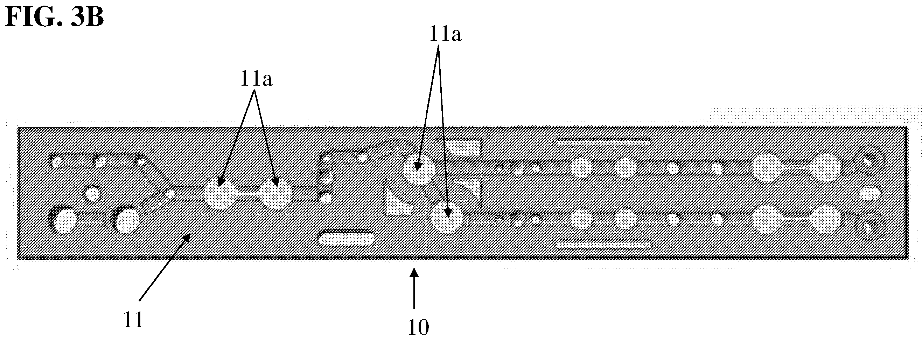

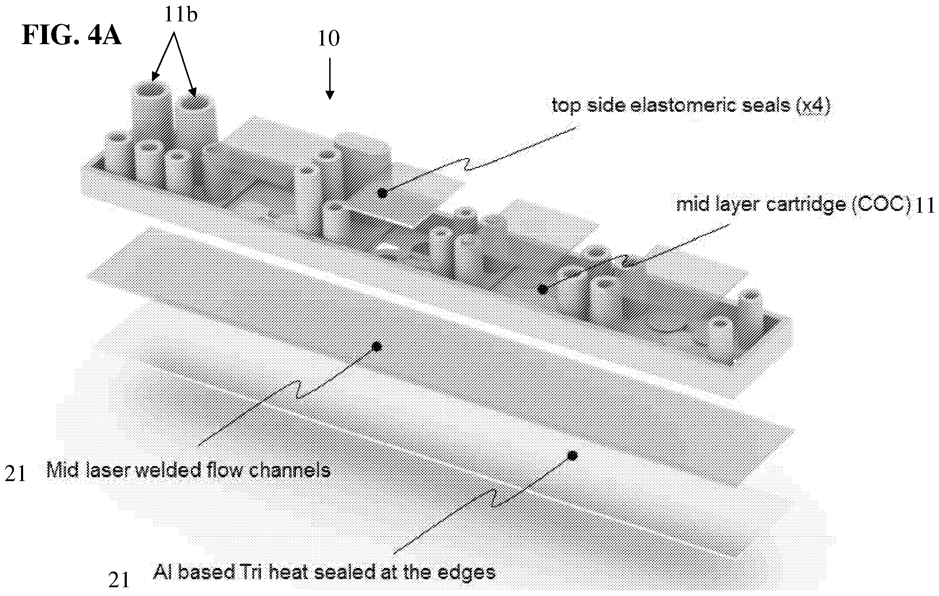

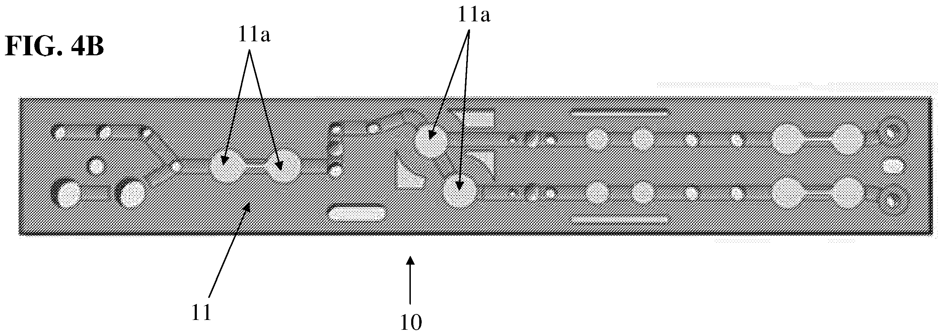

**FIG. 3A** is a representation of an example of the cartridge 10 using the configuration shown in **FIG. 1****,** and **FIG. 3B** is a schematic diagram showing the bottom surface of the first chassis 11 in such an example. **FIG. 4A** is an illustration of another example of the cartridge 10 using the configuration shown in **FIG. 1****,** and **FIG. 4B** is a schematic diagram showing the bottom surface of the first chassis 11 in such an example.

As generally illustrated in these figures and discussed in greater detail later herein, the first chassis 11 can have openings 11a; encapsulated reagents can be positioned in the openings 11a, and/or one or more elastomeric seals can be positioned on the first chassis 11 over the openings 11a. The encapsulated reagents can access the adjacent section of the channel and/or can be accessed from the adjacent section of the channel. The first chassis 11 can comprise one or more pots 11b positioned above one or more of the openings 11a, and reagents within the one or more pots 11b can access the adjacent section of the channel and/or can be accessed from the adjacent section of the channel through the corresponding opening 11a.

The fluid can be housed initially in a holding chamber (e.g., a foil "blister") by a barrier membrane. For example, the barrier membrane can be a weaker weld or seal between the first foil layer 31 and the first chassis 11 and can be positioned within the unsealed portions 31b of the first foil layer 31. In an embodiment, the barrier membrane can be spaced a distance inward from the intersection of the sealed portions 31a with the unsealed portions 31b of the first foil layer 31. The barrier membrane can be broken to allow the fluid therein to initiate formation of the channel.

Pressure, such as pressure from a piston pushed down within an adjacent pot 11b and/or pressure from an adjacent section of the compliant layer 20 pushed upward into the first foil layer 31, can break the barrier membrane and allow the fluid to exit the holding chamber and initiate formation of the channel. The breakable barrier membrane and process for breaking it is discussed in greater detail below.

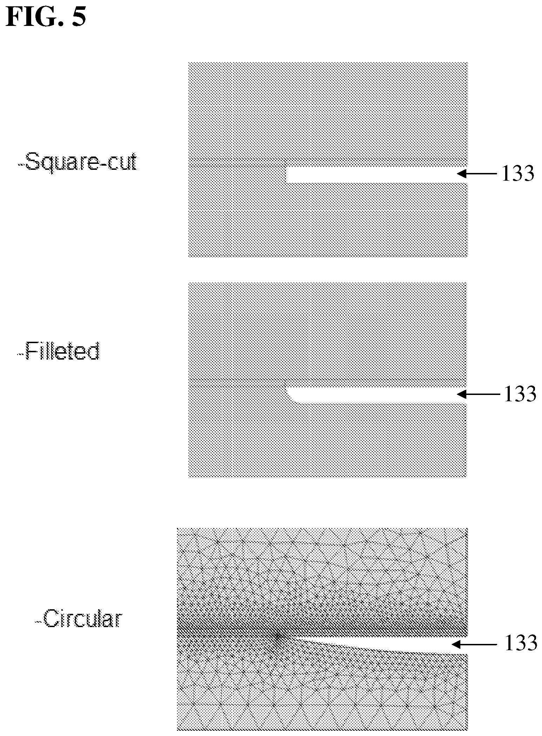

**FIG. 5** shows detailed views of the shape of the side of the groove 133 in the support block 12. Alternatively or additionally, the groove 133 can be positioned in the first chassis 11. In a preferred embodiment, the side of the groove 133 has a circular shape and can enable a cylindrical channel (bottom panel). Where beads such as magnetic beads are employed (discussed in more detail later herein), a filleted profile is preferred (middle panel).

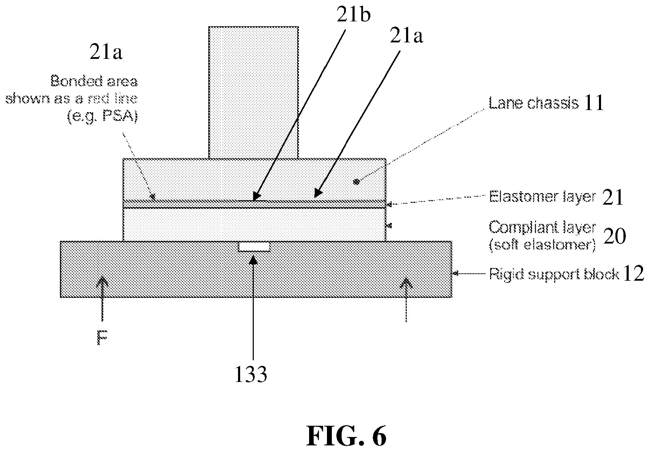

**FIG. 6** generally illustrates another configuration provided by the present disclosure. A first elastomeric membrane 21 can be positioned under the first chassis 11 and can comprise sealed portions 21a that are thermally welded, laser welded, bonded by pressure-sensitive adhesive, or otherwise fixedly attached to the first chassis 11. As a non-limiting example, the first elastomeric membrane 21 can have a thickness from about 25 µm to about 200 µm.

The compliant layer 20 can be positioned under the first elastomeric membrane 21. The compliant layer 20 can reversibly move between a closed position abutting the first elastomeric membrane 21 and an open position out of contact from the first elastomeric membrane 21, for example, by moving the support block 12 to which the compliant layer 20 can be fixedly attached.

The sealed portions 21a of the first elastomeric membrane 21 can circumscribe unsealed portions 21b of the first elastomeric membrane 21. The sealed portions 21a of the first elastomeric membrane 21 can thus define a periphery of the unsealed portions 21b of the first elastomeric membrane 21 such that the distance between the sealed portions 21a in any particular direction is the width of the unsealed portions 21b in the same direction.

The unsealed portions 21b can be continuous with each other such that they are configured to form the channel. Fluid, for example fluid containing one or more PCR reagents, can be directed between the first chassis 11 and a section of the unsealed portions 21b of the first elastomeric membrane 21. The fluid can be urged onto an adjacent downstream unsealed portion 21b of the first elastomeric membrane 21, for example by positive displacement and preferably without pneumatic displacement, thereby pushing the downstream unsealed portion 21b of the first elastomeric membrane 21 away from the first chassis 11. Consequently, the unsealed portions 21b of the first elastomeric membrane 21 can be sequentially pushed away from the first chassis 11 to form a channel between the first elastomeric membrane 21 and the first chassis 11.

The sealed portions 21a of the first elastomeric membrane 21 can thus define a periphery of the channel formed between the first chassis 11 and the unsealed portions 21b of the first elastomeric membrane 21; for example, if the sealed portions 21a of the first elastomeric membrane 21 are about 2 mm apart, the channel can have a width of about 2 mm. As discussed above and illustrated in **FIG. 38****,** a plurality of actuators within the instrument 8 (in one embodiment, toggle clamps) can be disposed along the channel to permit and restrict fluid from flowing along the fluid flow path. It should be appreciated that any suitable known actuator can be used to create valves along the elastomeric membranes and the fluid flow path. In discussions of the embodiments of the present invention having the first and second elastomeric membranes that act to define a fluid flow path, any disclosed valving can be accomplished using the toggle clamps of **FIG. 38** or other known actuation elements.

As the fluid progresses downstream, the upstream unsealed portions 21b of the channel which the fluid has evacuated can return to the resting state in which the first elastomeric membrane 21 is substantially flat. In a preferred embodiment, the compliant layer 20 is pushed into the unsealed portions 21b which contain the fluid to positively displace the fluid downstream and close the channel behind the fluid, for example by pushing the unsealed portions 21b containing the fluid to be substantially flat. If the upstream unsealed portions 21b are also held substantially flat by the compliant layer 20, the fluid can travel downstream.

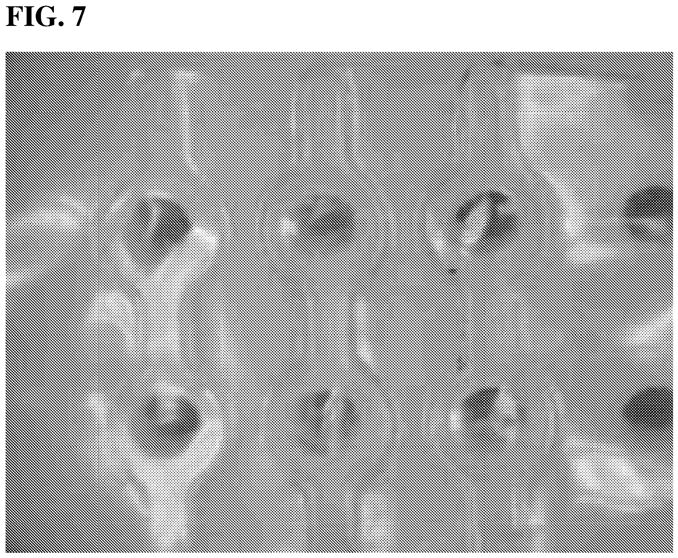

**FIG. 7** shows a photograph of a non-limiting example of a thermal plastic sheet sealing in an embodiment of the cartridge 10. In this embodiment, a low-density polyethylene (LDPE) sheet is used as a barrier to achieve a bond to a high-density polyethylene (HDPE) substrate.

### Reagent Storage and Introduction

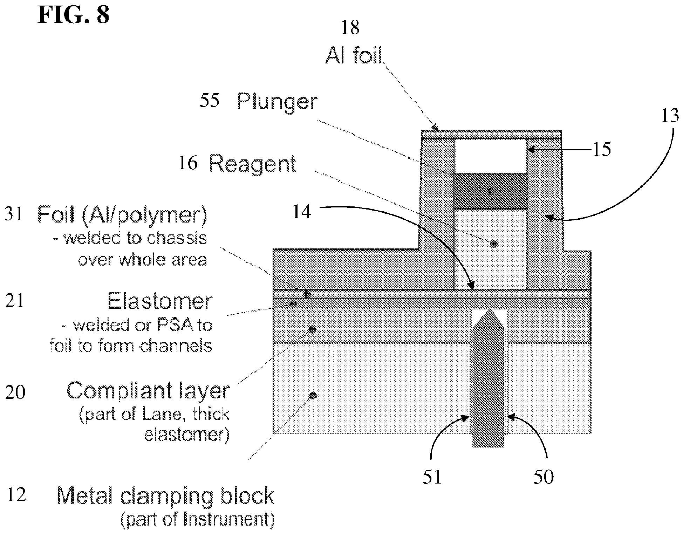

In various embodiments of the present disclosure, the reagents required for user-prescribed processes are stored within the cartridge, and either reconstituted, lyophilized, or simply added to the progressing sample in proper order during prep. As shown in **FIG. 8****,** an embodiment of the cartridge 10 comprises a foil membrane 18 covering one of the pots 11b, namely a first pot 13, in the first chassis 11. The first pot 13 can contain one or more reagents 16, for example one or more reagents for PCR and/or DNA sequencing. The first pot 13 can be a structure having a lower opening 14 and an upper opening 15 on an opposite end of the first pot 13 from the lower opening 14. The upper opening 15 of the first pot 13 can be sealed by the foil membrane 18 to prevent contamination of the interior of the first pot 13. In various embodiments, the foil membrane 18 also acts as a barrier to minimize fluid loss through a bung. It should be appreciated that, in several embodiments, the bung is an optional member that acts upon a plunger within a pot. In such an embodiment, the bung is depressed by an external force, pushing upon the associated plunger within the pot, which then forces the one or more reagents 16 through a newly established fluid pathway between the membranes of the fluid flow path.

In a preferred embodiment, the first pot 13 has a cylindrical shape, but the first pot 13 is not limited to a specific shape. Furthermore, any number of first pots 13 can be used, and the cartridge 10 is not limited to a specific number of the first pot 13.

The first foil layer 31 can be welded to the first chassis 11 to seal the one or more reagents 16 in the first pot 13. The first elastomeric membrane 21 can be positioned under the first foil layer 31. At least a portion of the upper surface of the first elastomeric membrane 21 can abut the first foil layer 31. The first elastomeric membrane 21 can be thermally welded to the first foil layer 31, laser-welded to the first foil layer 31, attached to the first foil layer 31 by a pressure sensitive adhesive, or affixed to the first foil layer 31 using any other suitable means known to the skilled artisan.

The compliant layer 20 can be positioned under the first elastomeric membrane 21, and at least a portion of the upper surface of the compliant layer 20 can abut the first elastomeric membrane 21, preferably the entirety of the upper surface of the compliant layer 20. The compliant layer 20 is preferably thicker than the first elastomeric membrane 21, for example at least twice as thick.

A lower passage 50 through the support block 12 and the compliant layer 20 can be positioned for a first lower plunger 51 to burst the first foil layer 31 without breaching the first elastomeric membrane 21. For example, the first lower plunger 51 can move upward in the lower passage 50 to push a vertically aligned section of the first elastomeric membrane 21 against a vertically aligned section of the first foil layer 31, thereby breaking the vertically aligned section of the first foil layer 31.

The first foil layer 31 in various embodiments is comprised of a foil sheet covering the entirety of the first elastomeric membrane 21. In all embodiments, a second elastomeric membrane 22 is situated adjacent to the first elastomeric membrane 21. The first elastomeric membrane 21 can include one or more predefined openings, perforations, or points of weakness, each aligning with one or more associated pots 11b. As discussed in more detail throughout this disclosure, the first and second membranes 21,22 can be connected together to form a fluid pathway. In various embodiments, the opening in the first elastomeric membrane 21 provides an entry point for fluid or other material to enter the fluid pathway from the one or more associated pots 11b. In one dual-membrane embodiment, the second elastomeric membrane 22 is continuous and includes no openings.

In an embodiment, a foil layer or a foil patch is aligned with and attached near each opening in the first elastomeric membrane 21. Some embodiments include individual foil patches covering each respective first elastomeric membrane opening, and other embodiments include one or more larger foil sheets that cover two or more first elastomeric membrane openings. The foil layer 31 can be welded over each individual first elastomeric membrane opening.

In embodiments with one or more foil layers sealed to the first elastomeric membrane 21, the material of the foil layer 31 is chosen to enable a controlled breaking upon activation of one or more lower plungers 51. In various embodiments, the foil layer 31 is made of a material more brittle than that of the second elastomeric membrane 22. For each first elastomeric membrane opening, upon activation with one or more lower plungers 51, the second elastomeric membrane 22 flexes and transfers pressure to the more brittle foil layer 31 disposed on the first elastomeric membrane opening until the foil layer 31 reaches a breaking point. Upon reaching a threshold pressure level, the plunger 51 causes the foil layer 31 to exceed its breaking point, and the fluid in a pot 11b associated with the first elastomeric membrane opening is fluidly communicable with the fluid pathway defined between the first and second elastomeric membranes 21,22 below the foil layer 31. Due to the selection of material for the first and second elastomeric membranes 21,22, the threshold pressure level that causes the foil layer 31 to reach its breaking point is insufficient to also cause the second elastomeric membrane 22 to be broken, pierced, or otherwise compromised. The second elastomeric membrane 22 will be flexed, but not broken. In various embodiments, the first and second elastomeric membranes 21,22 have the same or similar flexible properties. In alternative embodiments, the second elastomeric membrane 22 may have a different tolerance for flexion than the first elastomeric membrane 21.

The first pot 13 can comprise a first upper plunger 55 that can be moved downward in the first chassis 11, for example by being pressed upon by another component such as a piston. The first upper plunger 55 can thereby force the one or more reagents 16 through the broken section of the first foil layer 31.

The above-noted features, along with other reagent storage features discussed later herein, allow the cartridge 10 to advantageously provide independent storage of multiple different reagents and washes on-board a single lane of the cartridge 10. Moreover, the cartridge 10 prevents cross-contamination and excessive fluid loss during reagent storage.

### Thermal Processing and Mixing of Reagents

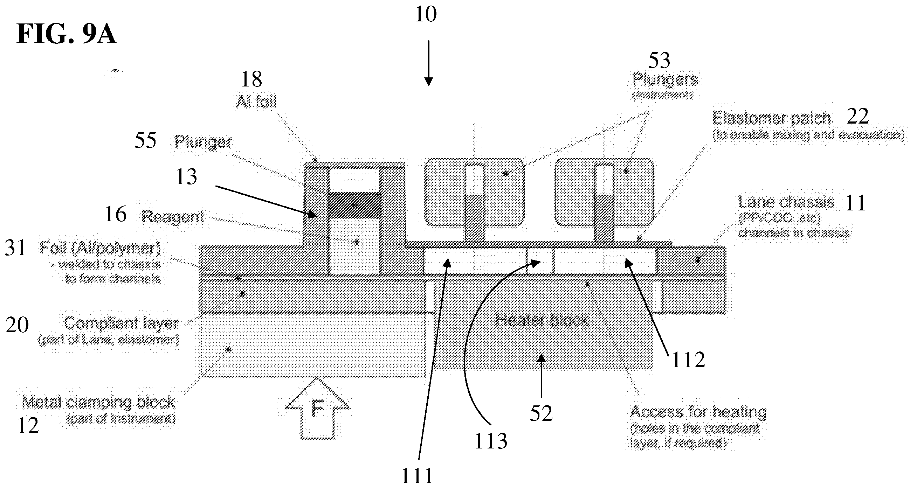

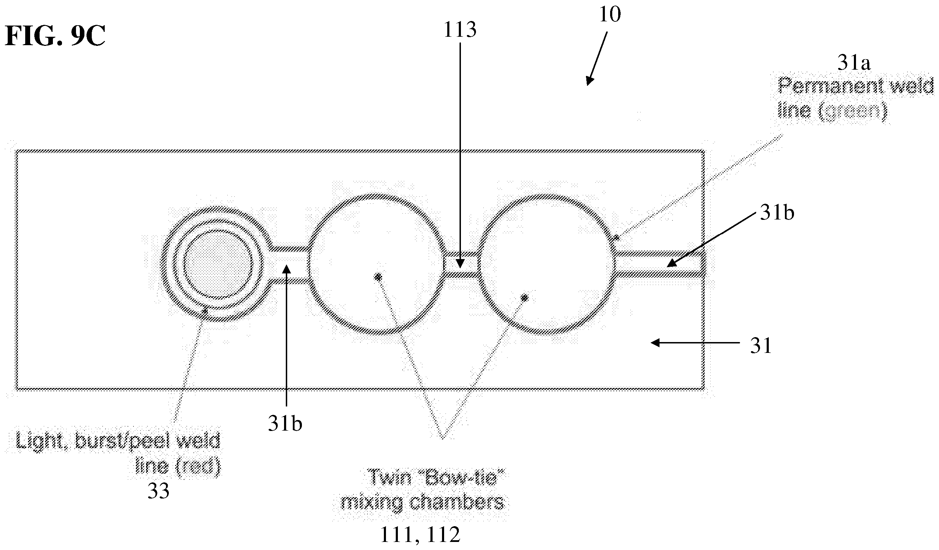

**FIGS. 9A-9D** generally illustrate an embodiment of the cartridge 10 which utilizes the structure depicted in **FIG. 1****.** As shown in **FIG. 9C****,** the one or more reagents 16 can be restricted from flowing downstream in the cartridge 10 by a barrier membrane 33. This embodiment can be employed by peeling or piercing the foil membrane 18, depressing the first upper plunger 55 to actuate the one or more reagents 16, and then the fluid pressure during the dispensing of the one or more reagents 16 can break the barrier membrane 33.

As shown in **FIG. 9A****,** the cartridge 10 and/or the instrument 8 in which the cartridge 10 is used can subject the one or more reagents 16 to thermal processing. For example, the instrument 8 in which the cartridge 10 is used comprises a heater block 52. Inserting the cartridge 10 into the instrument 8 can position the heater block 52 downstream from the barrier membrane 33.

The heater block 52 can be configured to heat the one or more reagents 16 when the one or more reagents 16 are positioned adjacent the heater block 52 (e.g., above or below). In some embodiments, the heater block 52 extends through holes in the compliant layer 20.

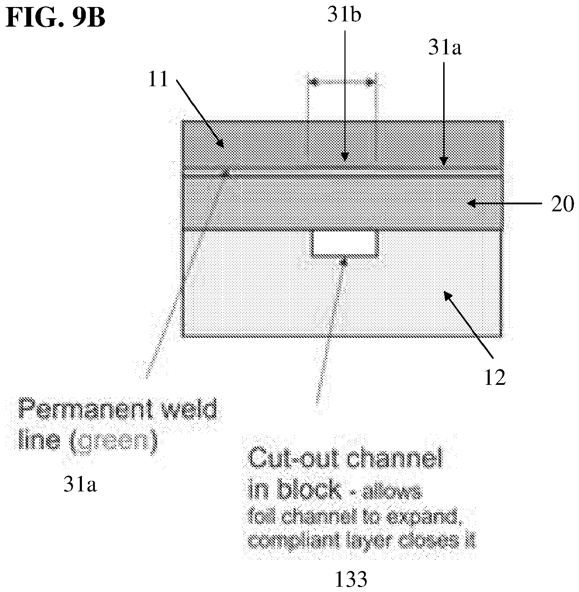

As discussed above, the unsealed portions 31b of the first foil layer 31 can form a channel when pushed away from the first chassis 11 by the one or more reagents 16. The unsealed portions 31b can be configured so that the channel directs the one or more reagents 16 to the position adjacent the heater block 52. As shown in **FIG. 9B****,** the support block 12 can comprise the groove 133 that allows the unsealed portions 31b of the first foil layer 31 to form the channel, and the channel is then closed by the compliant layer 20.

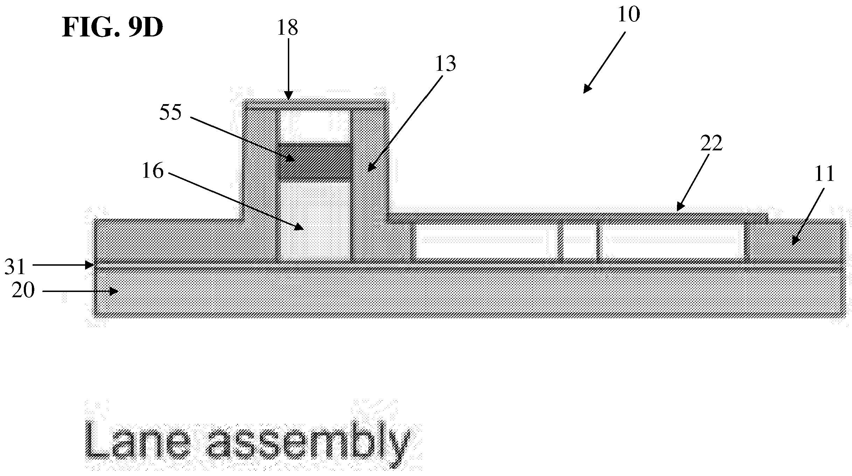

Referring to **FIGS. 9A****,** **9C** and **9D****,** the cartridge 10 comprises a first mixing chamber 111 and a second mixing chamber 112, and the first and second mixing chambers 111,112 can be positioned above or below the heater block 52. The first and second mixing chambers 111,112 are connected to each other by one or more mixing channels 113 that have a width smaller than the width of the chamber preceding the first mixing chamber 111 and/or the width of the channel subsequent to the second mixing chamber 112, for example less than half in relation thereto.

As shown in **FIG. 9A****,** a second elastomeric membrane 22 can be positioned over openings in the first chassis 11 and on an opposite side of the openings from the heater block 52. The first and second mixing chambers 111,112 can be at least partially formed by the second elastomeric membrane 22 and the first foil layer 31. One or both of the first and second mixing chambers 111,112 can be deformable to facilitate mixing of the one or more reagents 16 therein and/or to enable the one or more reagents 16 therein to be pumped through the cartridge 10.

For example, the instrument 8 in which the cartridge 10 is used comprises pistons 53 that reversibly depress and release the first mixing chamber 111 and/or the second mixing chamber 112 to control fluid flow. The first mixing chamber 111 can be depressed to urge the one or more reagents 16 therein into the one or more mixing channels 113 and/or the second mixing chamber 112. As a result, the cartridge 10 can provide on-board pumps that facilitate less complexity and easier use in the system (e.g. the instrument 8 can have a simpler design). **FIGS. 9A****,** **10A****,** **11A** and **12A** textually identify the component labeled with reference numeral 53 as "Plungers (Instrument)", while this detailed description refers to this component as piston(s) 53.

A portion of the compliant layer 20 reversibly closes the section of the channel upstream from the first and second mixing chambers 111,112 and thus function as a first valve. Another portion of the compliant layer 20 reversibly closes the section of the channel downstream from the first and second mixing chambers 111,112 and thus function as a second valve.

If the first valve prevents access to the unsealed portions 31b of the first foil layer 31 that are upstream from the first mixing chamber 111, the first mixing chamber 111 can be depressed by a corresponding piston 53 to urge the one or more reagents 16 from the first mixing chamber 111 into the one or more mixing channels 113 and/or the second mixing chamber 112. If the second valve prevents access to the unsealed portions 31b of the first foil layer 31 that are subsequent to the second mixing chamber 112, the second mixing chamber 112 can be depressed by a corresponding piston 53 to urge the one or more reagents 16 therein back into the one or more mixing channels 113 and/or the first mixing chamber 111 (preferably with the first mixing chamber 111 released). These steps can be repeated to achieve mixing of the one or more reagents 16 with each other and any additional reagents added thereto.

If the unsealed portions 31b of the first foil layer 31 that are subsequent to the second mixing chamber 112 are accessible and the first mixing chamber 11 is depressed by a corresponding piston 53, the second mixing chamber 112 can be depressed to urge the one or more reagents 16 from the second mixing chamber 112 into these unsealed portions 31b of the first foil layer 31 to form a channel therein.

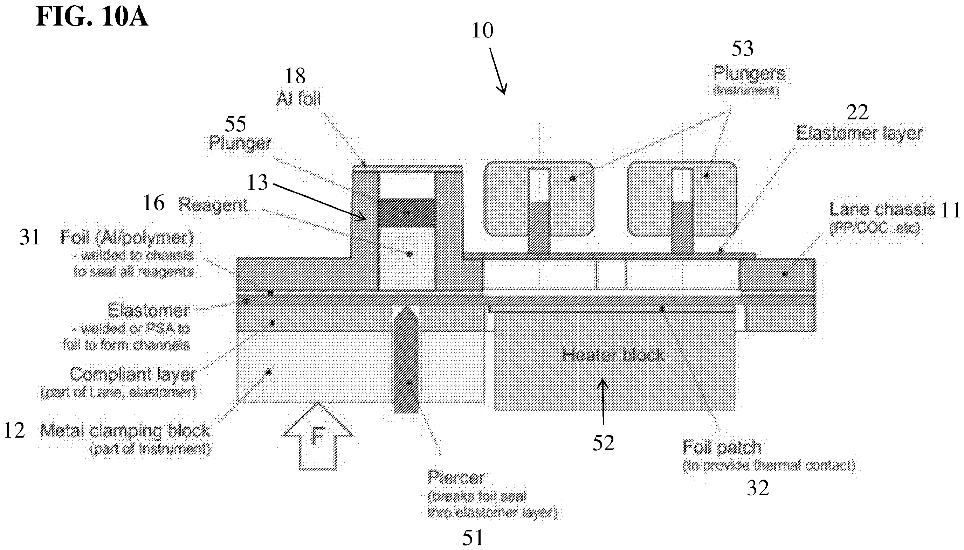

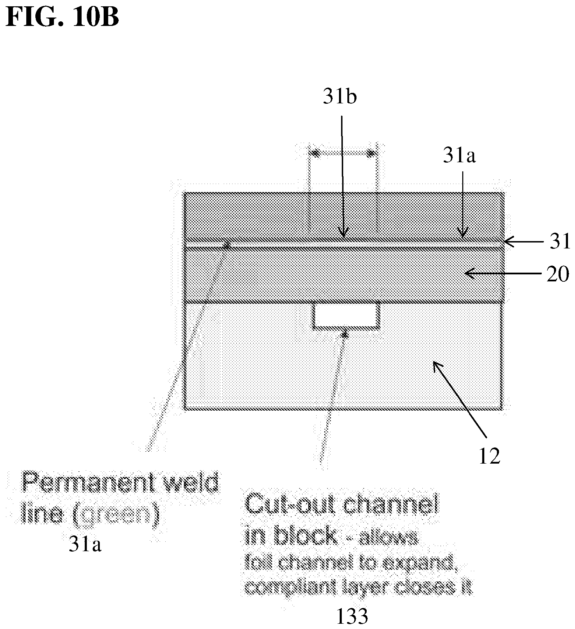

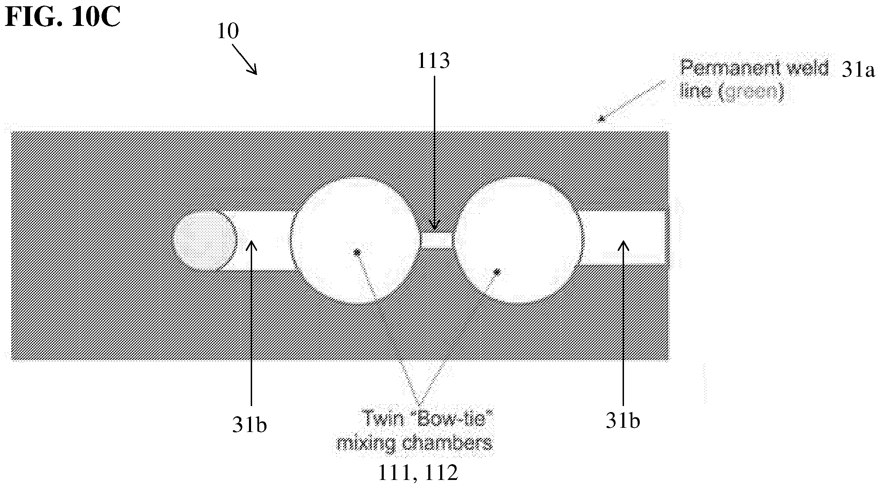

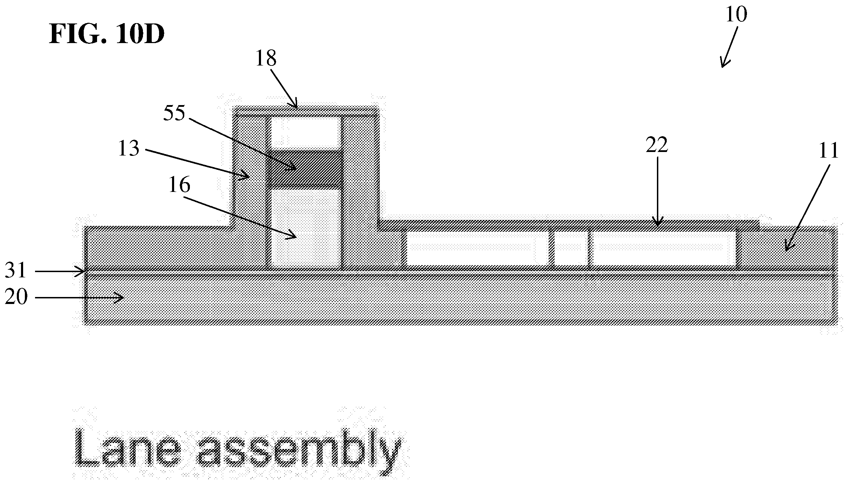

It should be appreciated that **FIGS. 9A-9D****,** **FIGS. 10A-10D****,** **FIGS. 11A-11C** and **FIGS. 12A-12C** illustrate similar respective views of different embodiments utilizing the structure depicted in **FIG. 8****.** Specifically, **FIGS. 10A-10D** generally illustrate an embodiment of the cartridge 10 that can have one or more components similar to those in the embodiment shown in **FIGS. 9A-9D****,** although preferably the first lower plunger 51 is utilized instead of the barrier membrane 33. **FIGS. 10A****,** **11A** and **12A** textually identify the component labeled with reference numeral 51 as "Piercer (breaks foil seal thru elastomer layer)", while this detailed description refers to this component as lower plunger(s) 51.

The first and second mixing chambers 111,112 are at least partially formed by the first and second elastomeric membranes 21,22. The embodiment in **FIGS. 10A-10D** can use a second foil layer 32 to provide thermal contact of the heater block 52 with the first and second mixing chambers 111,112, for example as a patch between the heater block 52 and the first elastomeric membrane 21.

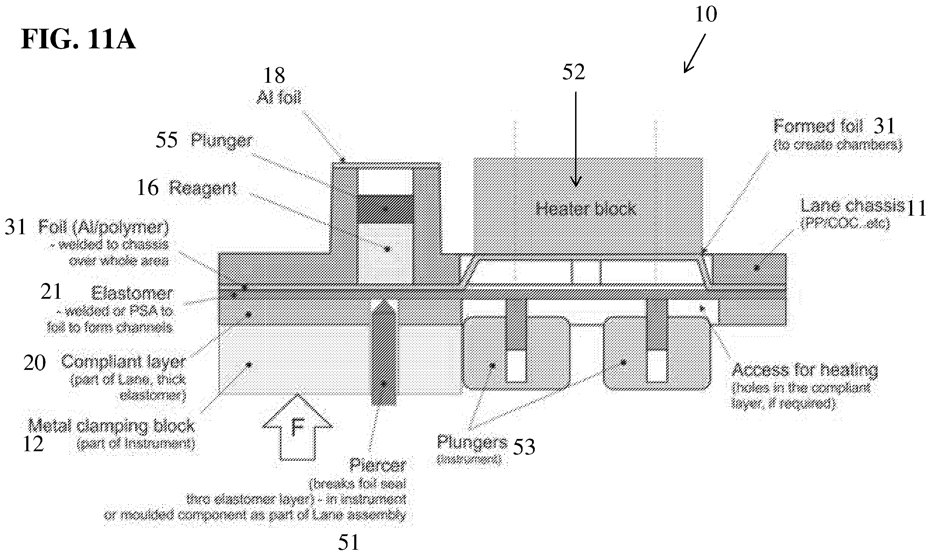

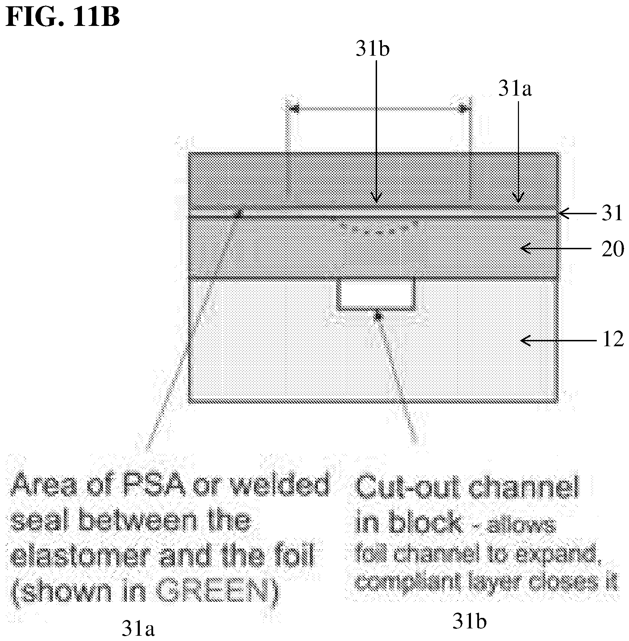

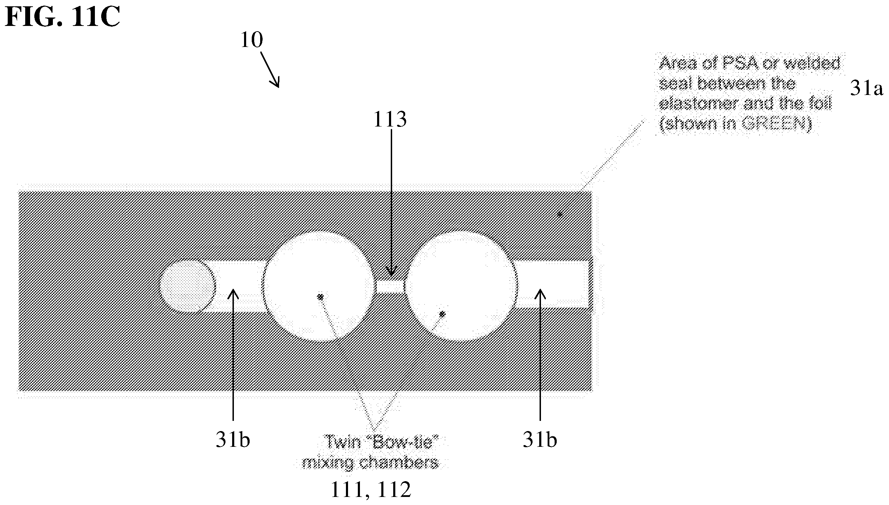

**FIGS. 11A-11C** generally illustrate an embodiment of the cartridge 10 that can have one or more components similar to those in the embodiment shown in **FIGS. 10A-10D****,** although preferably the first and second mixing chambers 111,112 are formed by the first elastomeric membrane 21 and the first foil layer 31.

The heater block 52 can be located above the first and second mixing chambers 111,112; and the pistons 53 that reversibly depress and release the first mixing chamber 111 and/or the second mixing chamber 112 can be located below the first and second mixing chambers 111,112. In such an embodiment, the first elastomeric membrane 21 can have holes that enable the movable components 53 to reversibly extend and retract therethrough.

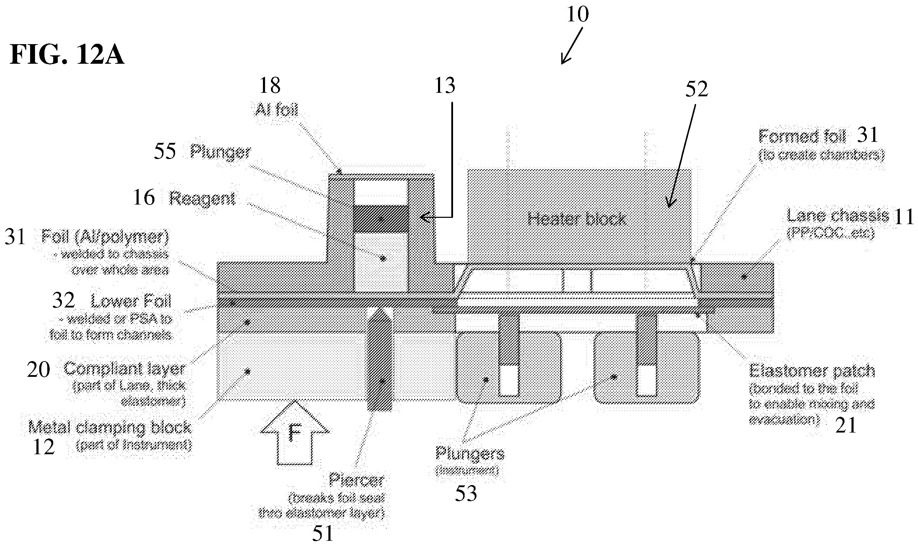

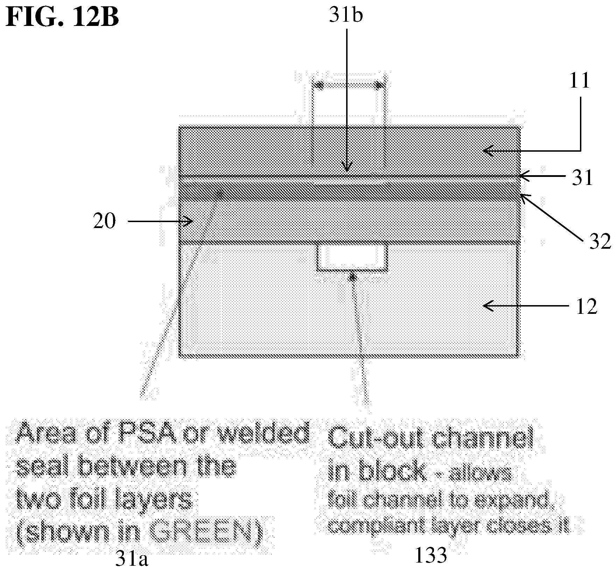

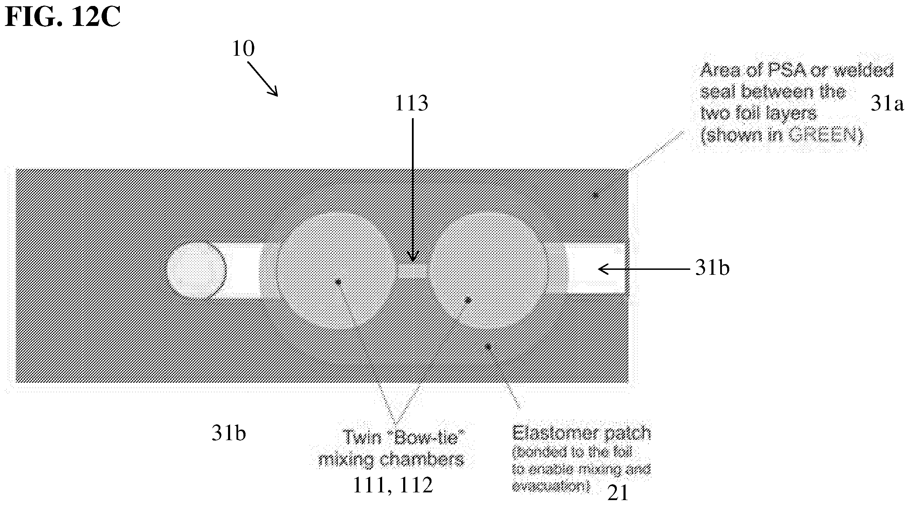

**FIGS. 12A-12C** generally illustrate an embodiment of the cartridge 10 which can have one or more components similar to those in the embodiment shown in **FIGS. 11A-11C****,** although preferably a second foil layer 32 is positioned between the first foil layer 31 and the compliant layer 20. The second foil layer 32 can be attached to the first foil layer 31 by welding, pressure sensitive adhesive, or any other means known to the skilled artisan. The first elastomeric membrane 21 can be in the form of a patch that enables the first and second mixing chambers 111,112 to undergo the fluid mixing discussed previously.

Preferably the upper surface of the first foil layer 31 is fixedly attached to the first chassis 11, preferably over substantially the entirety of the upper surface of the first foil layer 31. In this embodiment, the channel can form between the first and second foil layers 31,32. For example, the sealed portions 31a of the first foil layer 31 can be thermally welded, laser-welded, bonded by pressure-sensitive adhesive, or otherwise fixedly attached to portions 32b of the second foil layer 32. The first and second foil layers 31,32 comprise unsealed portions 31b,32b circumscribed by the sealed portions 31b,32b. The sealed portions 31a,32a of the first and the second foil layers 31,32 can thus define a periphery of the unsealed portions 31b,32b of the first and second foil layers 31,32 such that the distance between the sealed portions 31a,32a in any particular direction is the width of the unsealed portions 32a,32b in the same direction. The unsealed portions 32a,32b of the first and second foil layers 31,32 can be continuous such that they are configured to form the channel.

The one or more reagents 16 can be directed between the unsealed portions 31b,32b of the first and second foil layers 31,32. The fluid can be urged onto adjacent downstream unsealed portions 31b,32b of the first and second foil layers 31,32, for example by positive displacement and preferably without pneumatic displacement, thereby pushing the downstream unsealed portion 31b of the first foil layer 31 and the downstream unsealed portion 32b of the second foil layer 32 away from each other. Consequently, the unsealed portions 31b,32b of the first and second foil layers 31,32 can be sequentially pushed apart to form a channel between the first and second foil layers 31,32.

The sealed portions 31a,32a of the first and second foil layers 31,32 can thus define a periphery of the channel formed between the unsealed portions 3 1b,32b of the first and second foil layers 31,32. As the fluid progresses downstream, the upstream unsealed portions 31b,32b of the channel which the fluid has evacuated can return to the resting state in which the unsealed portions 31b,32b of the first and second foil layers 31,32 are substantially flat.

### Construction of Foil Layer and Elastomeric Membrane Defining Fluid Pathway

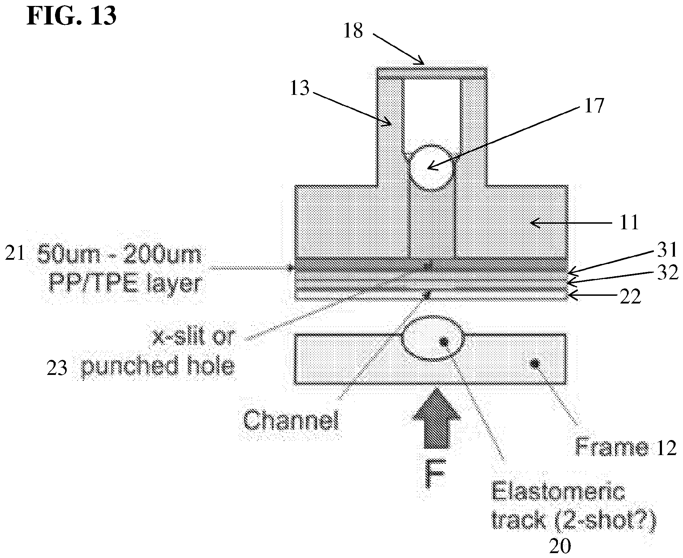

In an embodiment of the cartridge 10 generally illustrated in **FIGS. 13-16****,** the first elastomeric membrane 21 can be positioned under the first chassis 11, and at least a portion of the upper surface of the first elastomeric membrane 21 can abut the first chassis 11. The first foil layer 31 can be positioned under the first elastomeric membrane 21, and at least a portion of the upper surface of the first foil layer 31 can abut the first elastomeric membrane 21. The second foil layer 32 can be positioned under the first foil layer 31, and at least a portion of the upper surface of the second foil layer 32 can abut the first foil layer 31. The second elastomeric membrane 22 can be positioned under the second foil layer 32, and at least a portion of the upper surface of the second elastomeric membrane 22 can abut the second foil layer 32.

The compliant layer 20 can be positioned under the second elastomeric membrane 22, and the compliant layer 20 can reversibly move between a closed position abutting the second elastomeric membrane 22 and an open position out of contact with the second elastomeric membrane 22. The support block 12, which can be provided by the instrument 8 in which the cartridge 10 is used, can be positioned under the compliant layer 20; the support block 12 can move the compliant layer 20.

In this embodiment, the first elastomeric membrane 21 preferably is a layer of polypropylene/thermoplastic elastomer (PP/TPE) and preferably has a thickness of about 50 µm to about 200 µm, for example about 180 µm. The first foil layer 31 preferably is hard temper aluminum foil and preferably has a thickness of about 20 µm. The second foil layer 32 preferably is soft annealed aluminum foil and preferably has a thickness of about 25 µm. The second elastomeric membrane 22 preferably is a layer of polyurethane elastomer and preferably has a thickness of about 25 µm. Nevertheless, these components are not limited to a specific material or a specific thickness.

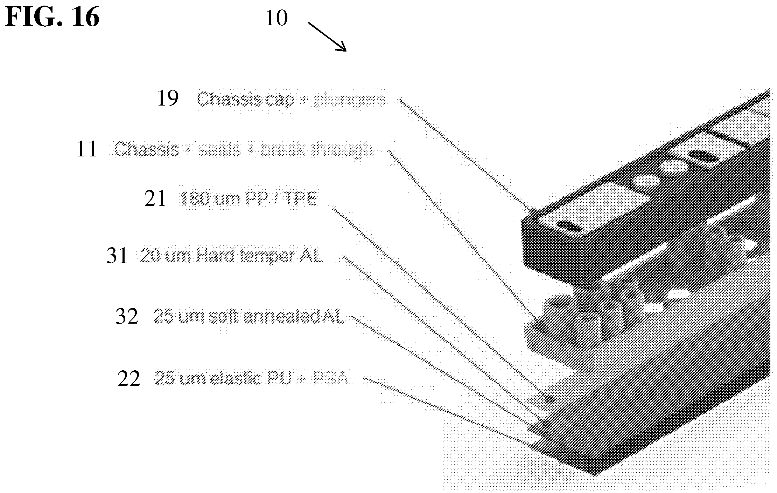

The first chassis 11 can comprise the first pot 13 that can contain the one or more reagents 16. The sample can be any size, as non-limiting examples about 10 µl, about 25 µl or about 50 µl. In the embodiment of the cartridge 10 shown in **FIG. 16****,** the cartridge 10 can comprise a cap 19 that connects to the first chassis 11 and covers the first pot 13.

Referring again to **FIGS. 13-15****,** the first pot 13 can contain a first ball 17 for urging the one or more reagents 16 through the lower opening 14. In a preferred embodiment, the first pot 13 has a passage extending from the upper opening 15 to the lower opening 14, and the first ball 17 is sized to move through at least part of the passage. Pushing the first ball 17 toward the lower opening 14 can push the one or more reagents 16 toward the lower opening 14 and through the lower opening 14. In an embodiment, the passage has a lower section with a narrower diameter than the first ball 17 to prevent the first ball 17 from reaching the lower opening 14. The first ball 17 is not limited to a specific shape, and the first ball 17 can be any component that can be moved within the passage to urge the one or more reagents 16 through the lower opening 14.

In an embodiment, the first ball 17 acts as the first upper plunger 55. In another embodiment, the first upper plunger 55 is a different component that is used to push the first ball 17.

The first elastomeric membrane 21 can comprise a first weakened portion 23, and the first pot 13 can be vertically aligned with the first weakened portion 23 of the first elastomeric membrane 21, such that the lower opening 14 of the first pot 13 is directly above the first weakened portion 23. The first weakened portion 23 can seal the lower opening 14 of the first pot 13 such that the one or more reagents 16 is held within the first pot 13, and then the first weakened portion 23 can be broken to allow the one or more reagents 16 to evacuate the first pot 13.

The first weakened portion 23 can be one or more slits, for example two slits intersecting each other to form an "X", a hole punched in the first elastomeric membrane 21; a section of the first elastomeric membrane 21 that has a smaller thickness than the adjacent sections of the first elastomeric membrane 21; or any structure that allows pressure applied to this section of the first elastomeric membrane 21 to break the first weakened portion 23 of the first elastomeric membrane 21 without damaging the adjacent portions of the first elastomeric membrane 21. The first weakened portion 23 is not limited to a specific structure.

In a preferred embodiment, the first weakened portion 23 is broken by the first lower plunger 51 being urged upward through the support block 12. The first lower plunger 51 can push upward against the section of the compliant layer 20 that is vertically aligned with the first lower plunger 51 such that the compliant layer 20 pushes against the first weakened portion 23 of the first elastomeric membrane 21 and thereby breaks the first weakened portion 23. Then the one or more reagents 16 in the first pot 13 can exit the first pot 13 through the broken first weakened portion 23, for example by using the first ball 17 and/or the first upper plunger 55.

As shown in **FIG. 14** and discussed briefly above, the first foil layer 31 and/or the second foil layer 32 can comprise one or more holes vertically aligned with the first weakened portion 23 and/or the first lower plunger 51. In such an embodiment, the one or more reagents 16 exiting the first pot 13 through the lower opening 14 can then travel through the broken first weakened portion 23 and the one or more holes of the first foil layer 31 and/or the second foil layer 32 to reach the upper surface of the second elastomeric membrane 22. The one or more reagents 16 which exited the first pot 13 to be positioned between the second foil layer 32 and the 2nd elastomeric membrane 22 can then proceed to downstream unsealed portions of the second foil layer 32 and the second elastomeric membrane 22, thereby forming the channel between the second foil layer 32 and the second elastomeric membrane 22. As discussed above, the fluid flow along the channel is controlled at least in part by the applied pressure of various actuators or clamps within the instrument 8 disposed along the channel. Such clamps can act as valves to selectively obstruct or permit passage of fluid as desired.

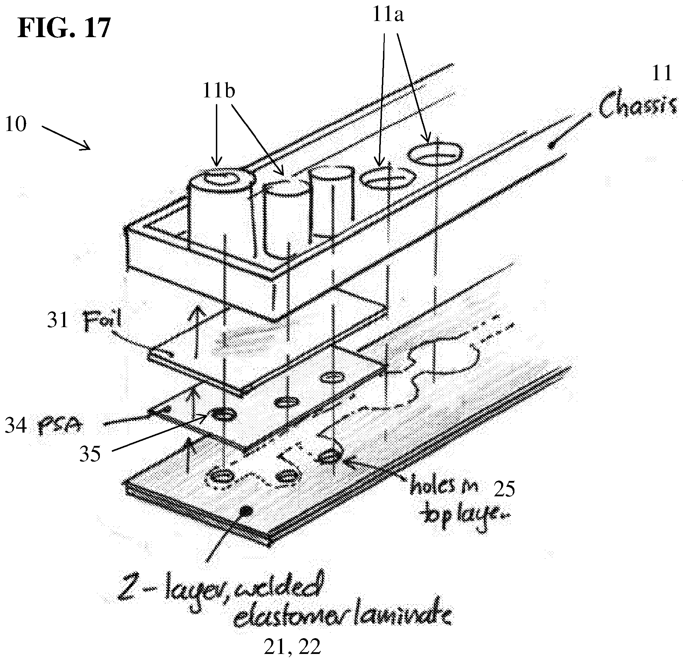

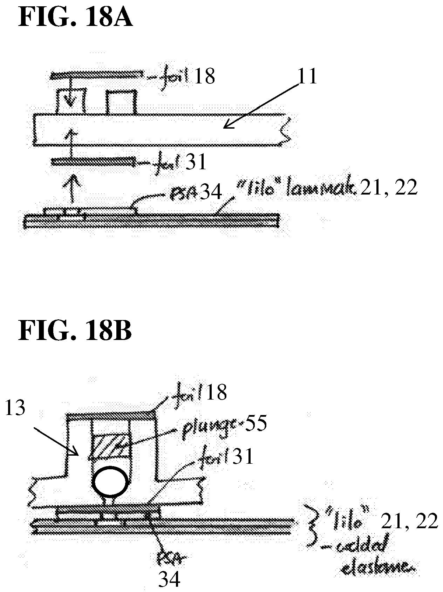

Assembly of the foil layers and elastomeric membranes can be accomplished in different ways. For example, as shown in **FIGS. 17****,** **18A****,** **18B****,** **19****,** **20A, 20B** and **21****,** various assembled configurations of the layers vis-à-vis the cartridge 10 and its other components are contemplated. In one such preferred embodiment, the first foil layer 31 can be positioned under the first chassis 11, and a pressure-sensitive adhesive (PSA) layer 34 can be positioned under the first foil layer 31. The first and second elastomeric membranes 21,22 can be positioned under the PSA layer 34 and above the compliant layer 20. As shown in **FIG. 18B****,** the first pot 13 can comprise the first ball 17 and/or the first upper plunger 55.

The first foil layer 31 can seal the lower opening 14 of the first pot 13. As discussed above, in some embodiments, the first foil layer 31 is provided as one or more patches that each cover only a portion of the lower surface of the first chassis 11, although in other embodiments the first foil layer 31 can be one or more continuous pieces that substantially covers the entirety of the lower surface of the first chassis 11.

In this preferred embodiment, the first elastomeric membrane 21 can have one or more holes 25, and the PSA layer 34 can have one or more holes 35. As shown in **FIG. 17****,** the holes 25,35 can be vertically aligned with the one or more pots 11b such that breaking the portion of the first foil layer 31 under the corresponding pot 11b can allow the one or more reagents 16 in the corresponding pot 11b to travel through the broken portion of the first foil layer 31, the one or more holes 35 of the PSA layer 34, and the one or more holes 25 of the first elastomeric membrane 21. As a result, the one or more reagents 16 can reach the upper surface of the second elastomeric membrane 22. The one or more reagents 16 which exited the first pot 13 to be positioned between the first and second elastomeric membranes 21,22 can then proceed to the downstream unsealed portions of the first and second elastomeric membranes 21,22.

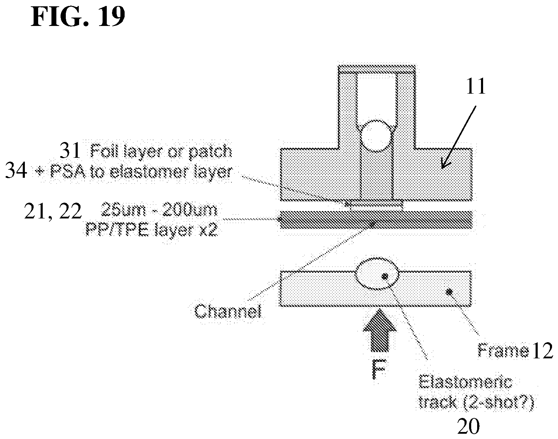

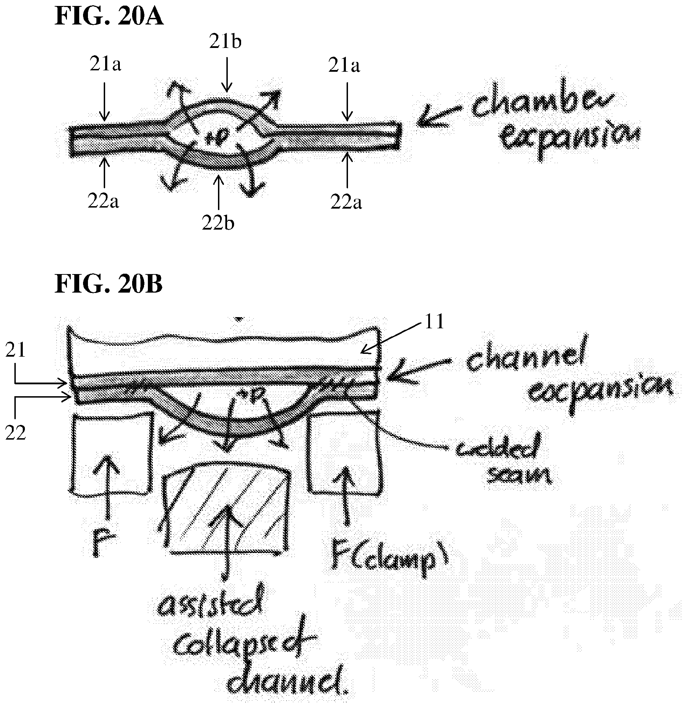

For example, as shown in **FIGS. 19****,** **20A** and **20B****,** the first elastomeric membrane 21 can comprise sealed portions 21a that are thermally welded, laser-welded, bonded by pressure-sensitive adhesive, or otherwise fixedly attached to portions 22a of the second elastomeric membrane 22. The first and second elastomeric membranes 21,22 comprise unsealed portions 21b,22b circumscribed by the sealed portions 21a,22a. The one or more reagents 16 can push an adjacent unsealed portion 22b of the second elastomeric membrane and the corresponding unsealed portion 21b of the first elastomeric membrane 21 away from each other to form the channel. The one or more reagents 16 can be urged between adjacent downstream unsealed portions 21b,22b of the first and second elastomeric membranes 21,22, for example by positive displacement and preferably without pneumatic displacement, thereby pushing the downstream unsealed portions 21b,22b of the first and second elastomeric membranes 21,22 apart. Consequently, the unsealed portions 21b,22b of the first and second elastomeric membranes 21,22 can be sequentially pushed apart to form the channel between the first and second elastomeric membranes 21,22.

The sealed portions 21a,22a of the first and second elastomeric membranes 21,22 can thus define a periphery of the channel; for example, if the sealed portions 21a,22a of the first and second elastomeric membranes 21,22 are about 2 mm apart, the channel can have a width of about 2 mm. As the fluid progresses downstream, the upstream portions of the channel which the fluid has evacuated can return to the resting state in which the first and second elastomeric membranes 21,22 are substantially flat, for example under pressure applied through the compliant layer 20 which forces the unsealed portion 21b of the first elastomeric membrane 21 and the unsealed portion 22b of the second elastomeric membrane 22 together to close the channel **(****FIG. 20B****).**

In this embodiment, a section of the unsealed portions 21b,22b of the first and second elastomeric membranes 21,22 forms a chamber **(****FIG. 20A****),** such as both of the first and second mixing chambers 111,112. The first part of the chamber can be formed by the first elastomeric membrane 21 and can be symmetrical to a second part of the chamber formed by the second elastomeric membrane 22. Preferably these chamber-forming sections abut or are proximate to each other before the one or more reagents 16 reach them, for example 0.0-50 µm apart, preferably 0.0-10 µm apart. The chamber can be formed by the one or more reagents 16 forcing the chamber-forming section of the first elastomeric membrane 21 apart from the corresponding chamber-forming section of the second elastomeric membrane 22. The chamber preferably has an ovoid shape but is not limited to a specific shape.

**FIG. 21** generally illustrates an embodiment of the cartridge 10, and in this embodiment the first and second membranes 21,22 can be configured as shown in **FIGS. 17****,** **18A****,** **18B****,** **19****,** **20A** and **20B****.** The first foil layer 31 can be positioned under one of the pots 11b of the chassis 11, for example a second pot 103. The PSA layer 34 can be positioned under the first foil layer 31, and the first and second elastomeric membranes 21,22 can be positioned under the PSA layer 34.

In a resting state, the unsealed portion 21b of the first elastomeric membrane 21 abuts or is proximate to the unsealed portion 22b of the second elastomeric membrane 22, for example 0.0-50 µm apart, preferably 0.0-10 µm apart. As discussed above, the one or more reagents 16 can be positively displaced between the unsealed portion 21b of the first elastomeric membrane 21 and the unsealed portion 22b of the second elastomeric membrane 22 in a region of the first and second elastomeric membranes 21,22 to push the unsealed portions 21b,22b away from each other in this region.

Then the fluid can be urged into an adjacent region of the first and second elastomeric membranes 21,22, for example by continued positive displacement, thereby pushing the unsealed portions 21b,22b apart in the adjacent region. Consequently, the unsealed portions 21b,22b can be sequentially pushed apart to form a channel between the first and second elastomeric membranes 21,22. As the fluid progresses through the unsealed portions 21b,22b, the upstream unsealed portions 21b,22b which the fluid has evacuated can return to the resting state in which they abut or are proximate to each other. Preferably, the channel does not contain any pressure sensitive adhesive, and thus the fluid does not contact such material.

For example, the unsealed portions 21b of the first elastomeric membrane 21 can face similarly dimensioned unsealed portions 22b of the second elastomeric membrane 22, and the unsealed portions 21b,22b can extend continuously from a point proximate to one end of the cartridge 10 to a point proximate to the opposite end of the cartridge 10 without being interrupted by the sealed portions 21a,22a.

Without being bound by theory, the present inventors believe that the fluid pushing the unsealed elastomeric membrane portions apart to a volume that substantially conforms to the volume of the fluid minimizes or prevents dead volume in the path travelled by the fluid and thus minimizes or prevents air bubbles in the fluid. In the devices provided by the present disclosure, the fluids can be confined between two elastomeric membranes; the chambers and the fluid paths can be defined by weld lines; zero-volume chambers and fluid channels can be achieved; sample insertion pressures can be used to inflate chambers and channels; and material performance can be assessed, for example during thermal cycling, by modeling the flow and the expansion.

Accordingly, the first and second elastomeric membranes 21,22 minimize or prevent dead volume in a channel traversed by a mesofluidic and/or microfluidic sample. Moreover, this configuration minimizes the number of fluidic connections in the cartridge 10 by providing a channel that continuously extends between processing steps without the need for transfer to another device and without the need for transfer into a different channel. For example, as discussed in greater detail hereafter, the cartridge 10 is capable of carrying out manual chamber processes, for example both magnetic bead handling and thermal cycling, and in some embodiments using similar materials and chamber designs for each process relative to the other processes.

As shown in **FIGS. 14** and **21****,** embodiments of the cartridge 10 can subject the one or more reagents 16 traversing the cartridge 10 to thermal processing. For example, the heater block 52, which can be provided by the instrument 8 in which the cartridge 10 is used, can be positioned downstream from the first pot 13. The heater block 52 can be configured to heat the one or more reagents 16 when the one or more reagents 16 is positioned between the first and second elastomeric membranes 21,22, above or below the heater block 52.

The unsealed portions 21b,22b of the first and second elastomeric membranes 21,22 can be configured to form a first chamber 24 above the heater block 52. The first chamber 24 preferably has an ovoid shape with a 0-75 µL volume and a height of about 0.0-2.0 mm, but the first chamber 24 is not limited to a specific shape or specific dimensions. The first chamber 24 is shown in **FIGS. 17** and **21** in an at least partially inflated state.

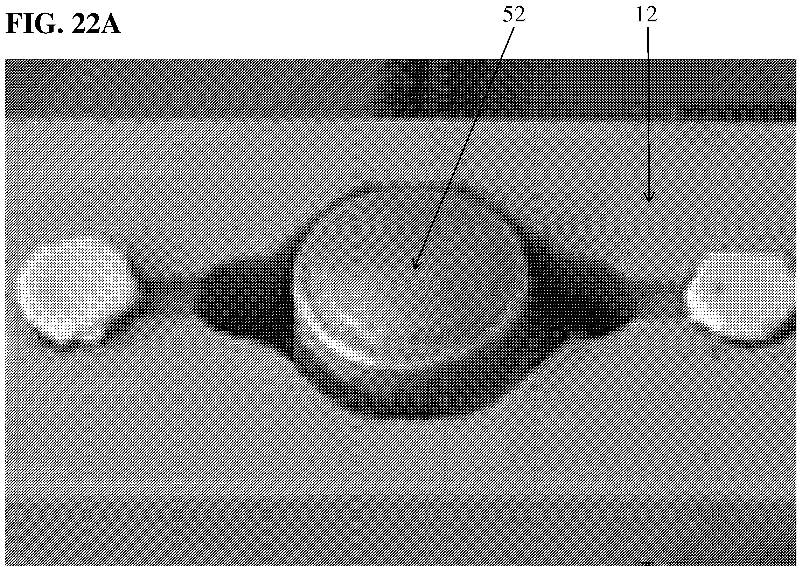

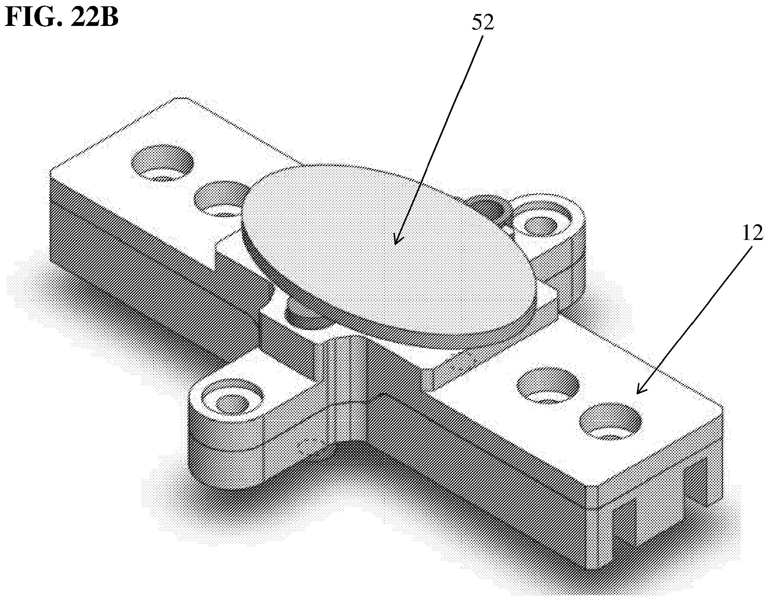

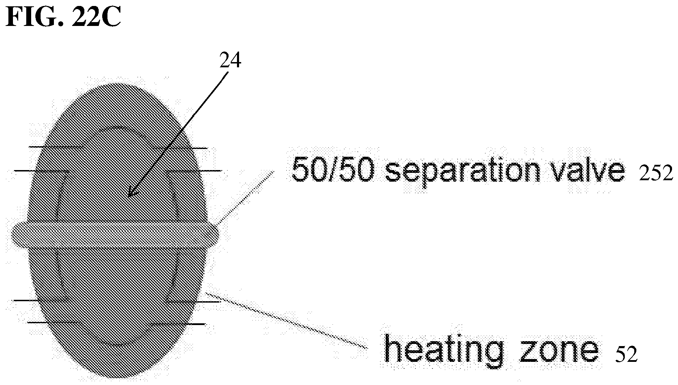

As generally illustrated in **FIG. 22A****,** the heater block 52 of one embodiment is convex, and the support block 12 is spaced apart from the lateral sides of the heater block 52 to reduce conduction and improve thermal cycling performance. As shown in **FIGS. 22B** and **22C****,** the two circular heater blocks which form the heater block 52 in the embodiment shown in **FIG. 17** are preferably merged into one oval block that forms the heater block 52 in the embodiment shown in **FIG. 21****.** In various alternative embodiments, the heater blocks 52 can have a flat, concave or convex contact surface.

**FIG. 22C** illustrates that the heater block 52 can extend past the edges of the first chamber 24 to reduce the heat conducted away from the first chamber 24 by the adjacent components of the cartridge 10. For example, the heater block 52 can have a length greater than the length of the first chamber 24 and/or a width greater than the width of the first chamber 24. The first chamber 24 can be a single chamber with an external separating valve 252.

Referring again to **FIGS. 14** and **21****,** preferably the first chamber 24 can be depressed to evacuate the first chamber 24 of the one or more reagents 16 and urge the one or more reagents 16 from the first chamber 24 to the downstream unsealed portions 21b,22b of the first and second elastomeric membranes 21,22. For example, one of the pistons 53 can depress the first chamber 24 to urge the one or more reagents 16 from the first chamber 24 to the downstream unsealed portions 21b, 22b of the first and second elastomeric membranes 21,22. In an embodiment, the pistons 53 can be pressed against one of the first elastomeric membrane 21 and the second elastomeric membrane 22 to push inward and thereby at least partially deflate the first chamber 24. As a result, the first chamber 24 can be used as a pump.

The one or more reagents 16 that have been thermally treated can travel downstream from the heater block 52 through the unsealed portions 21b, 22b of the first and second membranes 21,22. The cartridge 10 can comprise a second pot 103 that can contain at least a portion of a composition 116 to be mixed with the one or more reagents 16 that have been thermally treated. In an embodiment, the composition 116 comprises at least one reagent for amplification of DNA that is present in the one or more reagents 16 that have been thermally treated.

The second pot 103 can be a structure having a lower opening 114 and an upper opening 115 on an opposite end of the second pot 103 from the lower opening 114. In a preferred embodiment, the second pot 103 has a cylindrical shape, but the second pot 103 is not limited to a specific shape. Furthermore, any number of second pots 103 can be used, and the cartridge 10 is not limited to a specific number of the second pot 103. In the embodiment of the cartridge 10 shown in **FIG. 16****,** the cap 19 that can connect to the first chassis 11 can cover the second pot 103.

As discussed above, the various components of the cartridge enable a fluid passage of reagents from introduction through PCR and to clean-up. The cartridge facilitates superior sample prep by creating an integrated fluid pathway, through which the sample can travel from one process to another, and by incorporating mechanisms allowing external introduction of reagents throughout, as well as magnetic bead handling and thermal processing as necessary.

As discussed above, **FIGS. 14** and **21** illustrate various embodiments of the entire integrated workflow contemplated by the automated device of the present disclosure. In **FIGS. 14** and **21****,** the upper opening 115 of the second pot 103 can be sealed, for example with a foil membrane 128, to prevent contamination of the interior of the second pot 103. The second pot 103 can contain a second ball 118 for urging the composition 116 through the lower opening 114 of the second pot 103. In a preferred embodiment, the second pot 103 has a passage extending from the upper opening 115 to the lower opening 114, and the second ball 118 is sized to move through at least part of the passage. Pushing the second ball 118 toward the lower opening 114 can push the composition 116 toward the lower opening 114 and through the lower opening 114. In an embodiment, the passage has a lower section with a narrower diameter than the second ball 118 to prevent the second ball 118 from reaching the lower opening 114. The second ball 118 is not limited to a specific shape, and the second ball 118 can be any component that can be moved within the passage to urge composition 116 through the lower opening 114.

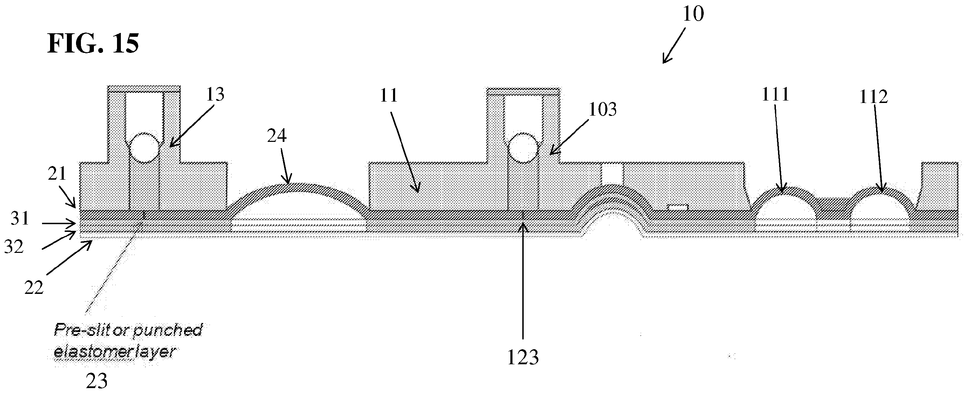

In the embodiment shown in **FIGS. 14** and **15****,** the lower opening 114 of the second pot 103 can be adjacent to or abut the first elastomeric membrane 21, for example in a sealing engagement. The first elastomeric membrane 21 can comprise a second weakened portion 123, and the second pot 103 can be vertically aligned with the second weakened portion 123 of the first elastomeric membrane 21 such that the lower opening 114 of the second pot 103 is directly above the second weakened portion 123. The second weakened portion 123 can seal the lower opening 114 of the second pot 103 such that the composition 116 is held within the second pot 103, and then the first weakened portion 23 can be broken to allow the one or more reagents 16 to evacuate the first pot 13.

The second weakened portion 123 can be one or more slits, for example two slits intersecting each other to form an "X", a hole punched in the first elastomeric membrane 21; a section of the first elastomeric membrane 21 that has a smaller thickness than the adjacent sections of the first elastomeric membrane 21; or any structure that allows pressure applied to this section of the first elastomeric membrane 21 to break the second weakened portion 123 of the first elastomeric membrane 21 without damaging the adjacent portions of the first elastomeric membrane 21. The second weakened portion 123 is not limited to a specific structure.

In a preferred embodiment, the second weakened portion 123 is broken by a second lower plunger 151 urged upward through the support block 12. The second lower plunger 151 can push upward against the section of the second elastomeric membrane 22 that is vertically aligned with the second lower plunger 151 such that the second elastomeric membrane 22 pushes against the second weakened portion 123 of the first elastomeric membrane 21 and thereby breaks the second weakened portion 123. Then the composition 116 in the second pot 103 can exit the second pot 103 through the broken second weakened portion 123.

The first foil layer 31 and/or the second foil layer 32 can comprise one or more holes vertically aligned with the second weakened portion 123 and/or the second lower plunger 151. In such an embodiment, the composition 116 exiting the second pot 103 through the lower opening 114 can then travel through the broken second weakened portion 123 and the one or more holes of the first and second foil layers 31, 32 to reach the upper surface of the second elastomeric membrane 22.

### Introduction of Solid Reagents

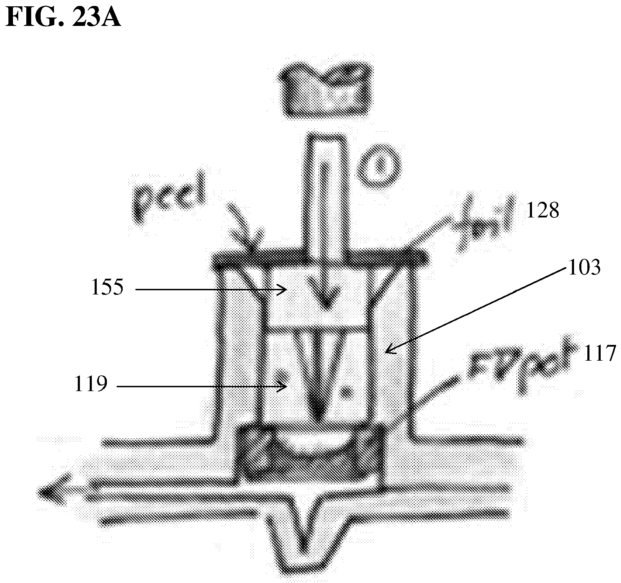

As shown in **FIGS. 23A-23C****,** the composition 116 can be at least partially formed from one or more solid reagents 117, and the solid reagents 117 can be positioned in the second pot 103. For example, the one or more solid reagents 117 can be a lyophilized bead. As another example, the one or more solid reagents 117 can be a freeze-dried cake, and the cake can be dried within the pot or dried between foil layers and a seal. Different foil layers can be used with the cake; in an embodiment, the upper foil layer is weaker than the lower foil layer to ensure that only the upper foil layer breaks when external force is applied and/or the upper foil layer breaks before the lower foil layer when external force is applied.

The composition 116 can be formed by reconstituting the lyophilized bead 117, for example by using diluent 119. Reconstitution of the lyophilized bead 117 by the diluent 119 can form the composition 116, and the composition 116 can mix with the one or more reagents 16 that have been thermally treated. At least a portion of the diluent 119 can be positioned in the second pot 103. The second pot 103 can comprise a second upper plunger 155, and the second upper plunger 155 can facilitate reconstitution of the lyophilized bead 117 by the diluent 119. As a result, the cartridge 10 is advantageously capable of on-board storage and reconstitution of dried reagents alongside wet reagents, unlike known mesofluidic and/or microfluidic processing devices.

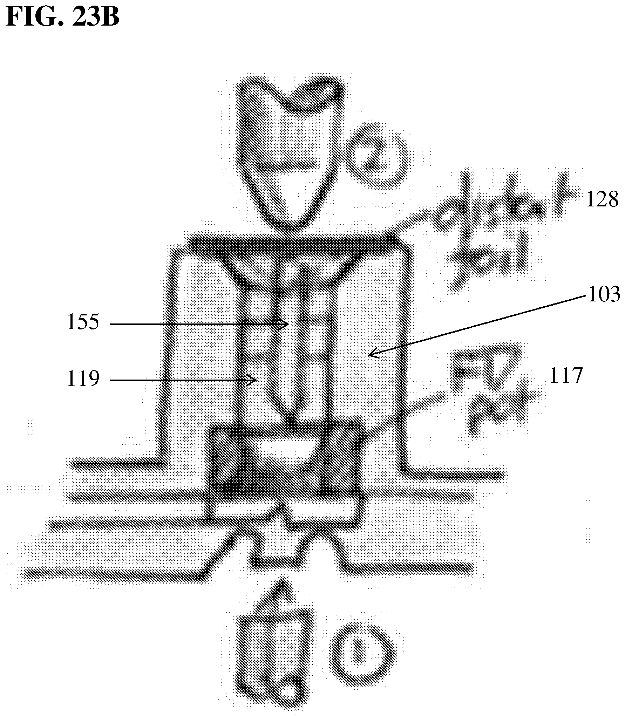

The lyophilized bead 117 can be covered by an upper membrane and a lower membrane. As shown in **FIGS. 23A** and **23B****,** the second upper plunger 155 can comprise a downward-pointing spike at a lower end of the second upper plunger 155. The second upper plunger 155 can be moved downward in the second pot 103, for example by being pressed by a bung, and thereby force the downward-pointing spike through the upper membrane, allowing the diluent 119 to reach the lyophilized bead 117.

As shown in **FIG. 23A****,** the second upper plunger 155 can comprise an extension on an upper end of the second upper plunger 155, and the extension can be depressed to move the second upper plunger 155 downward in the second pot 103. The downward-pointing spike can break the upper membrane, allowing the diluent 119 to reach the lyophilized bead 117, and can break the lower membrane, allowing the composition 116 formed by reconstitution of the lyophilized bead 117 by the diluent 119 to reach the one or more reagents 16 that has been thermally treated. The first and second elastomeric membranes 21, 22 can be configured to accommodate the downward-pointing spike.

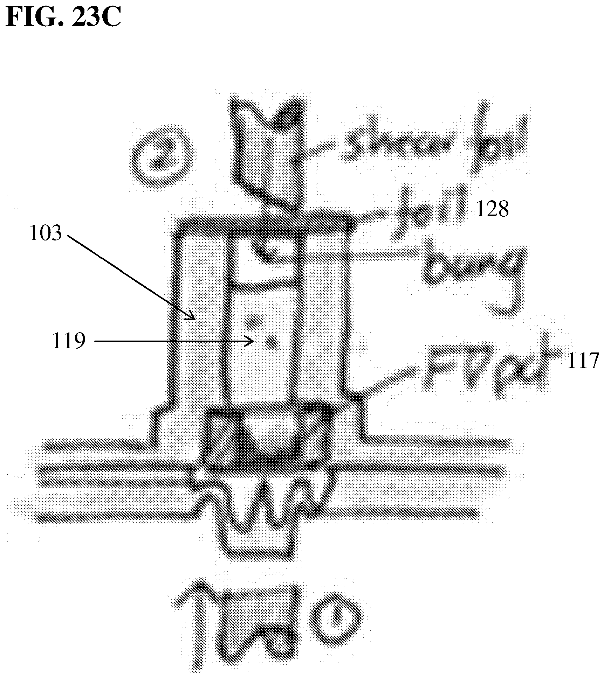

As shown in **FIGS. 23B** and **23C****,** a component (e.g. one or both of the first and second elastomeric membranes 21, 22) can comprise an upward-pointing spike. The upward-pointing spike can be pushed toward the second pot 103, for example by being pressed by a bung, to force the upward-pointing spike upward through the lower membrane covering the lyophilized bead 117. Preferably, the upward-pointing spike is employed and then the second upper plunger 155 such that the second upper plunger 155 pushes the composition 116 out of the second pot 103 through the opening created by the upward-pointing spike **(****FIG. 23B****).** In another embodiment shown in **FIG. 23C****,** the second upper plunger 155 can not have the downward-pointing spike, and the upward-pointing spike can puncture both the lower and upper membranes covering the lyophilized bead 117.

In the embodiment shown in **FIG. 21****,** the lower opening 114 of the second pot 103 can be adjacent to or abut the first foil layer 31, for example in sealing engagement. The first foil layer 31 can be positioned under a third pot 213, and the pressure-sensitive adhesive (PSA) layer 34 can be positioned under the first foil layer 31.

The second pot 103 can contain the diluent 119, and a third pot 213 can contain the lyophilized bead 117. The third pot 213 can be a structure having a lower opening 214 and an upper opening 215 on an opposite end of the third pot 213 from the lower opening 214. In a preferred embodiment, the third pot 213 has a cylindrical shape, but the third pot 213 is not limited to a specific shape. Furthermore, any number of third pots 213 can be used, and the cartridge 10 is not limited to a specific number of the third pot 213. In some embodiments, a third lower plunger 152 can be used to break the section of the first foil layer 31 adjacent to the lower opening 214 of the third pot 213.

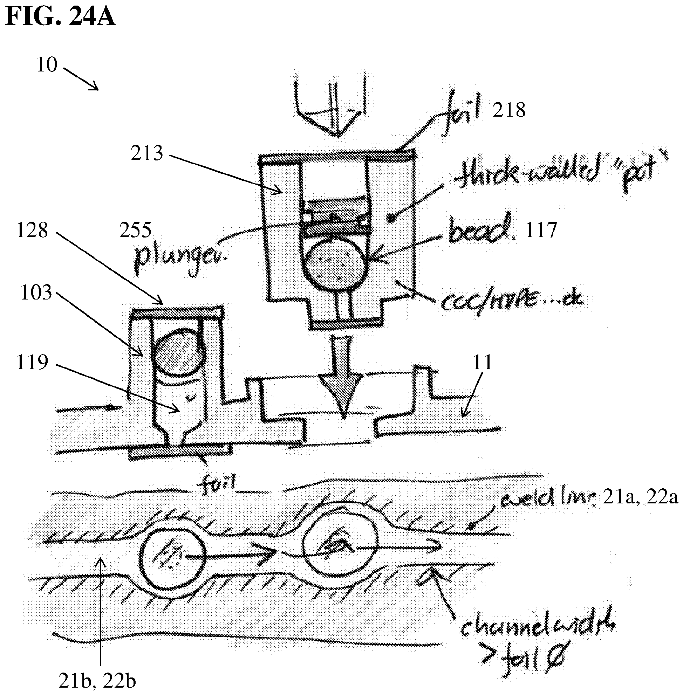

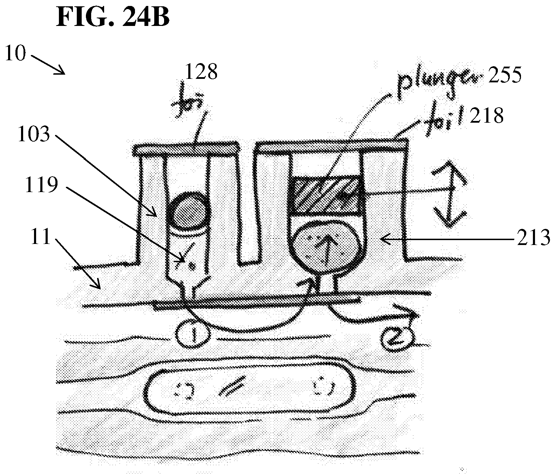

The third pot 213 can be a separate piece which connects to the first chassis 11 and thus can enable the cartridge 10 to be modular (e.g., a specific third pot 213 can be selected from a plurality of third pots 213 based on the desired material contained by the selected third pot 213). For example, a portion of the third pot 213 can have a shape that is similarly dimensioned to an opening in the first chassis 11 **(****FIG. 24A****).** Alternatively, the third pot 213 can be integral with the first chassis 11 such that at least a portion of the third pot 213 and at least a portion of the first chassis 11 are formed by one single piece of material **(****FIG. 24B****).**

The upper opening 215 of the third pot 213 can be sealed, for example with a foil membrane 218, to prevent contamination of the interior of the third pot 213. The third pot 213 can contain a third upper plunger 255 for urging the lyophilized bead 117 through the lower opening 214 after reconstitution of the lyophilized bead 117, as discussed in more detail hereafter. In a preferred embodiment, the third pot 213 has a passage extending from the upper opening 215 to the lower opening 214, and the third upper plunger 255 is sized to move through at least part of the passage. In an embodiment, the passage has a lower section with a narrower diameter than the third upper plunger 255 to prevent the third upper plunger 255 from reaching the lower opening 214.

The first elastomeric membrane 21 can have at least one of the one or more holes 25 vertically aligned with the lower opening 114 of the second pot 103 such that breaking the portion of the first foil layer 31 under the second pot 103 can allow the diluent 119 in the second pot 103 to travel through the broken portion of the first foil layer 31 and the one or more holes 25 of the first elastomeric membrane 21 to reach the upper surface of the second elastomeric membrane 22.

In a preferred embodiment, the PSA layer 34 can have one or more pre-formed holes. The portion of the first foil layer 31 under the second pot 103 is broken by the second lower plunger 151 which is urged upward through the support block 12. The second lower plunger 151 can push upward against the section of the first and second elastomeric membranes 21, 22 that is vertically aligned with the second lower plunger 151 such that the second elastomeric membranes 22 push against the portion of the first foil layer 31 under the second pot 103 and thereby breaks the portion of the first foil layer 31 under the second pot 103. Additionally or alternatively, the PSA layer 34 can have weakened structure which can be broken together with the portion of the first foil layer 31.

The first elastomeric membrane 21 and the PSA layer 34 can have at least one of the one or more holes 25 vertically aligned with the lower opening 214 of the third pot 213 such that breaking the portion of the first foil layer 31 under the third pot 213 can allow the diluent 119 which has exited the second pot 103 to travel through the one or more holes 25 of the first elastomeric membrane 21 and the PSA layer 34 and the broken portion of the first foil layer 31 to reach the interior of the third pot 213. The diluent 119 entering the third pot 213 can reconstitute the lyophilized bead 117. The third upper plunger 255 can be pushed downward to urge the composition 116 formed by reconstitution of the lyophilized bead 117 by the diluent 119 through the lower opening 214 of the third pot 213. The composition 116 can travel through the broken portion of the first foil layer 31 and the one or more holes 25 of the first elastomeric membrane 21 and the PSA layer 34 to reach the upper surface of the second elastomeric membrane 22.

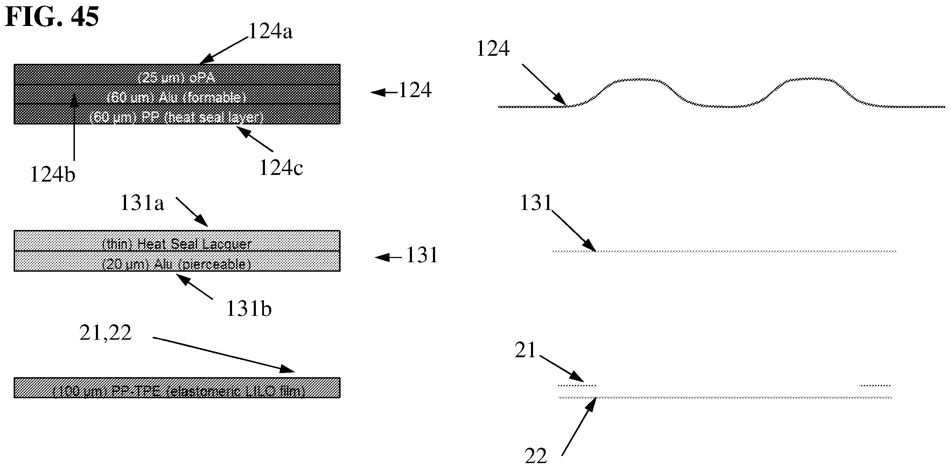

**FIGS. 45-48** generally illustrate another embodiment in which the one or more solid reagents 117 (e.g., a lyophilized bead) can be mixed with the one or more reagents 16 that are being processed or handled in the cartridge 10. As shown in **FIG. 45****,** the components of this embodiment can comprise one or more of: (i) a blister layer 124 that can comprise a layer of amine-terminated polyaniline (OPA) 124a, an aluminum layer 12b and a polypropylene layer 124c; (ii) a pierceable foil layer 131 that can comprise a heat seal lacquer 131a and a pierceable aluminum layer 131b; and, always, (iii) the first and second elastomeric membranes 21, 22.

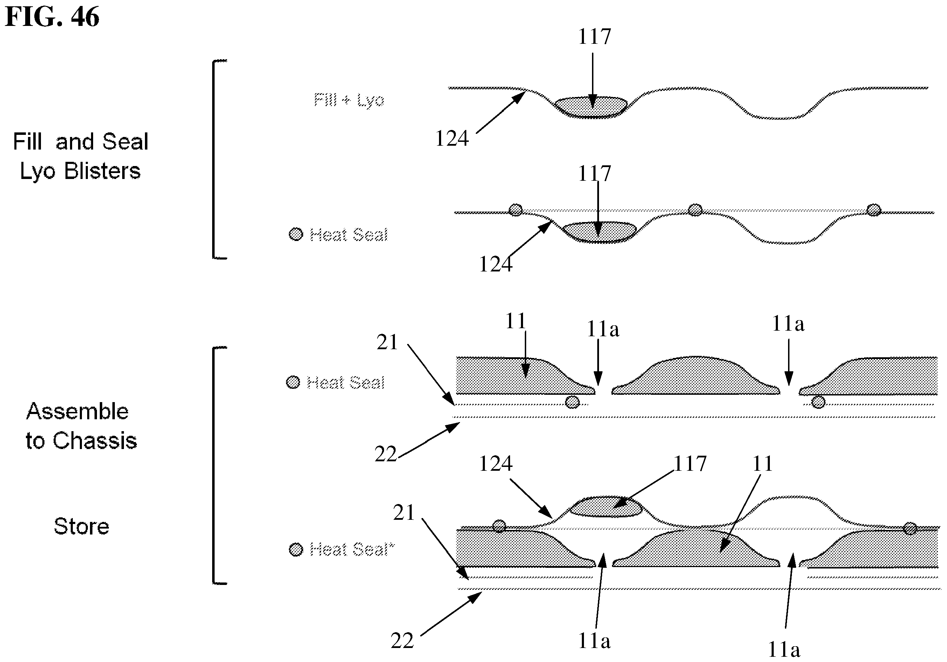

As shown in **FIG. 46****,** the one or more solid reagents 117 can be positioned in the blister layer 124, for example by filling the one or more solid reagents 117 into the blister layer 124 while the one or more solid reagents 117 are in liquid form and then lyophilizing the blister layer 124 with the one or more solid reagents therein. After the one or more solid reagents 117 are positioned in the blister layer 124, the pierceable foil layer 131 can be heat-sealed to the blister layer 124 to encapsulate the one or more solid reagents 117. Then, the blister layer 124 with the pierceable foil layer 131 thereon can be heat-sealed to the upper surface of the first chassis 11 to form the twin blisters.

The first and second elastomeric membranes 21, 22 can be heat-sealed to the bottom surface of the first chassis 11. Additionally or alternatively, a pressure-sensitive adhesive can be used to attach the first and second elastomeric membranes 21,22 to the bottom surface of the first chassis 11. The first and second elastomeric membranes 21,22 can be attached to the bottom surface of the first chassis 11 before the blister layer 124 with the pierceable foil layer 131 thereon is heat-sealed to the upper surface of the first chassis 11, after the blister layer 124 with the pierceable foil layer 131 thereon is heat-sealed to the upper surface of the first chassis 11, and/or during the blister layer 124 with the pierceable foil layer 131 thereon being heat-sealed to the upper surface of the first chassis 11.

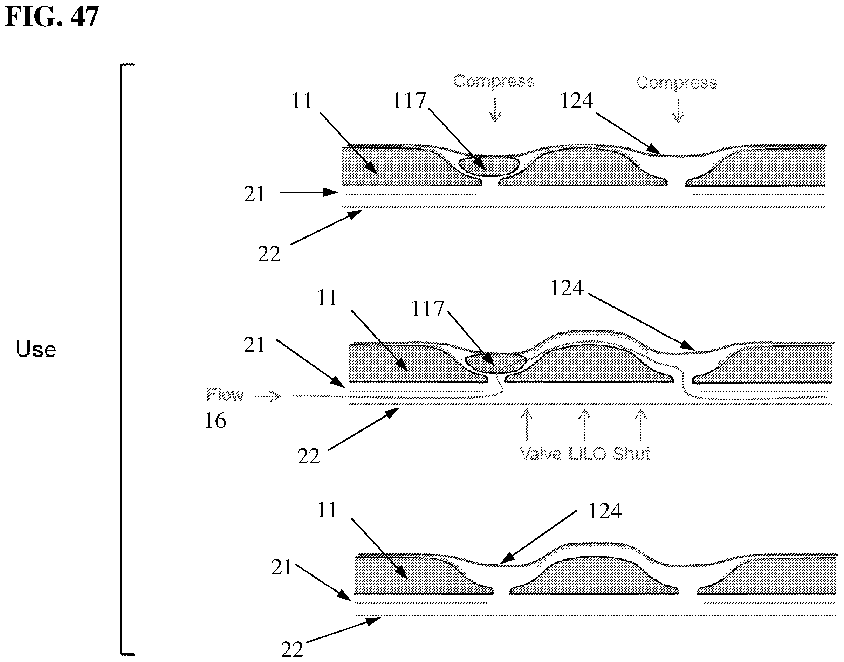

The one or more solid reagents 117 (e.g., a lyophilized bead) can be vertically aligned with the openings 11a in the first chassis 11 such that the one or more solid reagents 117 can be accessed by the one or more reagents travelling through the first and second elastomeric membranes 21,22. For example, as shown in **FIG. 47****,** the twin blister can be used by compressing the blisters such that the pierceable foil layer 131 is broken in the areas adjacent the openings 11a. The section of the first chassis 11 between the openings 11a can be valved close, for example by a clamp or another device that can press at least one of the first and second elastomeric membranes 21,22 against the first chassis 11, to prevent fluid flow therethrough. Then the one or more reagents 16 can travel through the first one of the openings 11a to reach the one or more solid reagents 117 and mix with the one or more solid reagents 117. The resultant mixture can travel between the blister layer 124 and the upper surface of the first chassis 11 and then go through the second one of the openings 11a to return to a position between the first and second elastomeric membranes 21,22.

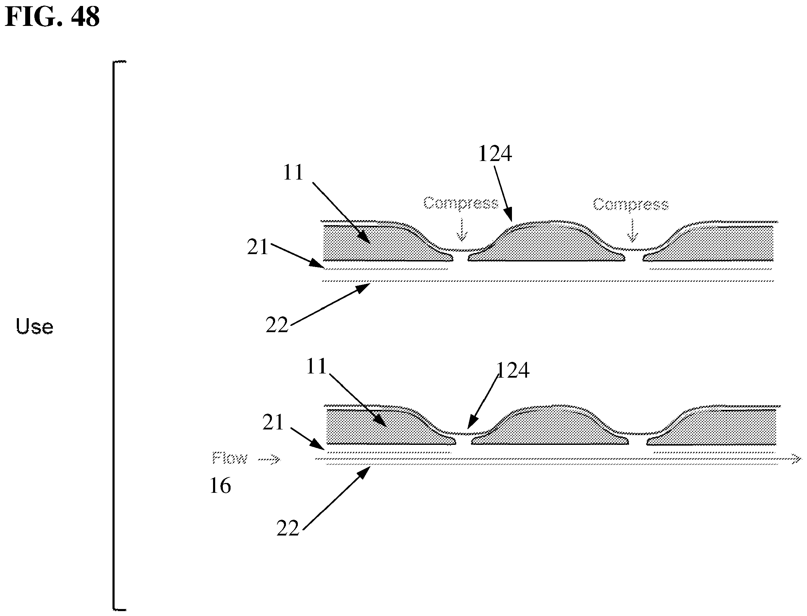

As shown in **FIG. 48****,** the cartridge 10 can be used without the one or more solid reagents 117 positioned thereon. For example, the areas of the blister layer 124 that cover the openings 11a can be compressed downward such that the one or more reagents 16 are maintained below the first chassis 11. The one or more reagents 16 can travel downstream by continuing along the second elastomeric membrane 22.

The twin blister embodiment generally illustrated in **FIGS. 45-48** can provide storage stability by fully enclosing the one or more solid reagents 117 (e.g., a lyophilized bead). Furthermore, the manufacturability can be advantageous because this embodiment is amenable to strip format processing and separates blister fabrication from chassis assembly. Moreover, this embodiment minimizes dead volume significantly due to blister compression.

As shown in **FIGS. 14** and **21****,** the one or more reagents 16 that has been thermally treated can be urged downstream through the unsealed portions 21b,22b of the first and second elastomeric membranes 21,22 so that the one or more reagents 16 that has been thermally treated mixes with the composition 116. Preferably the reagents in the composition 116 amplify the single-stranded DNA in the one or more reagents 16. The one or more reagents 16 that has been thermally treated can mix with the composition 116 within the unsealed portions 21b, 22b of the first and second elastomeric membranes 21,22.

### Magnetic Bead Handling Mechanism

As shown in **FIGS. 14** and **21****,** the cartridge 10 can comprise a magnetic bead handling mechanism 300 that can be configured to bind, wash and elute the amplified DNA. Thus the cartridge 10 can perform processes such as PCR and/or DNA sequencing without the need for a centrifuge. The magnetic bead handling mechanism 300 can comprise one or more magnets, for example an upper magnet 301 and/or a lower magnet 302, and the magnets 301,302 can be motor-driven. For example, a processor, optionally communicatively connected to a graphic user interface, can control movement of the magnets 301,302 (e.g. vertical movement to change a height of the magnet relative to the first and second elastomeric membranes 21,22; horizontal movement to change a distance of the magnet along the length and/or the width of the cartridge 10; and rotation movement of the magnet). Each of the magnets 301, 302 can be a permanent magnet and/or can be an electromagnet which can be activated, deactivated, and have its field strength adjusted, for example by the processor. Any number of magnets may be used, and disclosures herein regarding the first and second magnets 301,302 also apply to additional magnets, e.g. a third magnet, a fourth magnet, etc.

The magnetic bead handling mechanism 300 can comprise one or more chambers 310 formed by the first and second elastomeric membranes 21,22, and the one or more reagents 16 can enter the one or more chambers 310 after being mixed with the composition 116. One or more washes of the bead-bound DNA can be performed while the magnets 301, 302 maintain the position of the beads in the chambers 310, for example by the magnets 301, 302 keeping the bead-bound DNA clumped together. Preferably the magnets 301, 302 are held stationary while the beads are washed. In a preferred embodiment, the one or more chambers 310 comprise the first and second mixing chambers 111,112 and the one or more mixing channels 113 discussed previously herein.

The magnetic bead handling mechanism 300 can comprise temperature sensors and temperature control devices (not shown), which can be controlled by the processor in the instrument 8 in which the cartridge 10 is used.

In various alternative embodiments, dye exterminator beads may be employed to extract dye from the reagent before it continues in the process. In some embodiments, such beads can be non-magnetic dye sponges encapsulated in a chamber along the flexible membrane, accessible via a pierceable bypass valve. In some embodiments, the non-magnetic dye exterminator beads are employed instead of the magnetic bead handling mechanism discussed above. In other embodiments, the non-magnetic dye exterminator beads are used as a supplement to the magnetic bead handling mechanism.

Referring again to **FIG. 21****,** the compliant layer 20 and the support block 12 can form one or more valves at various positions of the cartridge 10. A closed position of the valve can close a section of the channel formed by the unsealed portions 21b,22b of the first and second elastomeric membranes 21,22 and force the fluid in the channel upstream and/or downstream (depending on the position of the other valves and pistons). An open position of the valve can allow a section of the channel to open under pressure from the one or more reagents 16.

For example, a closed position of the valve can hold a section of the unsealed portions 21b of the first elastomeric membrane 21 against the corresponding (e.g., vertically aligned) section of the unsealed portions 22b of the second elastomeric membrane 22. Consequently, the one or more reagents 16 cannot move into or through these sections of the unsealed portions 21b,22b of the first and second elastomeric membranes 21,22. The open position of the valve can allow the section of the unsealed portions 21b of the first elastomeric membrane 21 to move away from the corresponding section of the unsealed portions 22b of the second elastomeric membrane 22 when the one or more reagents 16 reaches these sections.

As a non-limiting example, one of the valves can be positioned between the first pot 13 and the heater block 52, can be in a closed position to prevent the one or more reagents 16 injected from the first pot 13 from reaching the first chamber 24 adjacent the heater block 52, then can be in an open position to allow the one or more reagents 16 to reach the first chamber 24 adjacent the heater block 52, and then can return to the closed position to prevent the one or more reagents 16 in the first chamber 24 adjacent the heater block 52 from travelling upstream in the cartridge 10 (e.g., back toward the first pot 13).

As another non-limiting example, one of the valves can be positioned between the heater block 52 and the second pot 103, can be in a closed position to prevent the one or more reagents 16 in the first chamber 24 adjacent the heater block 52 from reaching the second pot 103, then can be in an open position to allow the one or more reagents 16 to reach the second pot 103, and then can return to the closed position to prevent the one or more reagents 16 adjacent the second pot 103 from travelling upstream in the cartridge 10 (e.g., back toward the first chamber 24 above the heater block 52).

As yet another non-limiting example, one of the valves can be positioned between the third pot 213, can be in a closed position to prevent the one or more reagents 16 adjacent the third pot 213 from reaching the magnetic bead handling mechanism 300, then can be in an open position to allow the one or more reagents 16 to reach the magnetic bead handling mechanism 300, and then can return to the closed position to prevent the one or more reagents 16 adjacent the magnetic bead handling mechanism 300 from travelling upstream in the cartridge 10 (e.g., back toward the third pot 213).

As shown in **FIGS. 14** and **21****,** the one or more chambers 310 of the magnetic bead handling mechanism 300 can comprise the first mixing chamber 111, the second mixing chamber 112, and the one or more mixing channels 113 that provide fluid communication between the first and second mixing chambers 111,112.

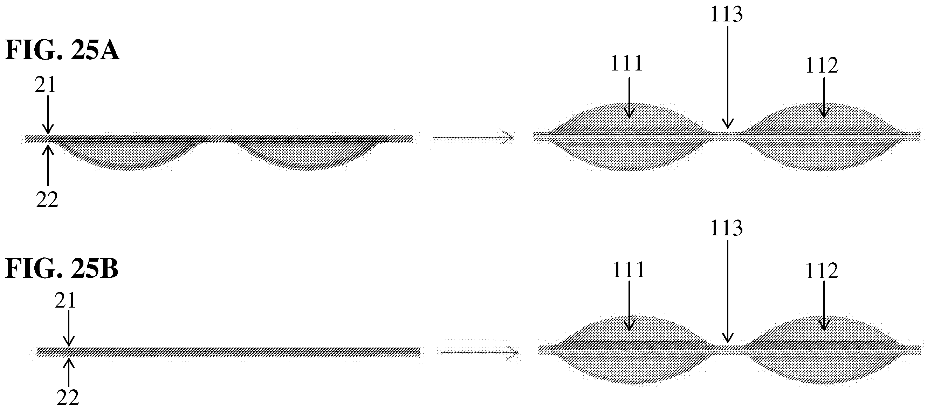

In an embodiment, the sections of the first and second elastomeric membranes 21,22 that correspond to the first and second mixing chambers 111,112 can be pre-formed with the desired shape **(****FIG. 25A****).** In such an embodiment, before the one or more reagents 16 reaches these pre-formed sections of the first and second elastomeric membranes 21,22, the pre-formed section of the first elastomeric membrane 21 can curve downward and abut the pre-formed section of the second elastomeric membrane 22. Then, when the one or more reagents 16 reaches these sections, the one or more reagents 16 can push the pre-formed section of the first elastomeric membrane 21 away from the pre-formed section of the second elastomeric membrane 22 such that the first and second mixing chambers 111,112 are formed. Advantages of such an embodiment can include ease of inflation of the first and second mixing chambers 111,112 and a minimized pressure inside the first and second mixing chambers 111,112.

In another embodiment, the sections of the first and second elastomeric membranes 21,22 that correspond to the first and second mixing chambers 111,112 are flat before the one or more reagents 16 reaches these sections **(****FIG. 25B****).** Then, when the one or more reagents 16 reaches these sections, the one or more reagents 16 can push them apart such that the first and second mixing chambers 111,112 are formed. Advantages of such an embodiment can include improved thermal contact and a reduced chance of air bubbles inside the first and second mixing chambers 111,112.

In other embodiments, one or both of the first and second mixing chambers 111,112 are not pre-formed sections of the first and second elastomeric membranes 21,22.

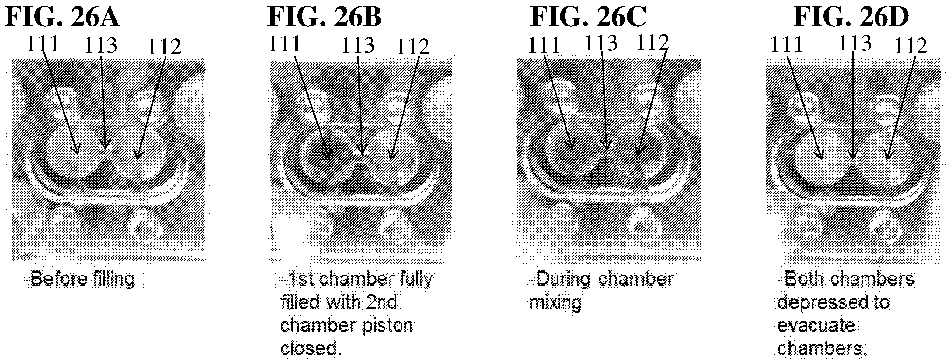

**FIGS. 26A-26D** are photographs from an initial chamber fluid handling test performed in an embodiment in which the first and second mixing chambers 111,112 comprise pre-formed sections of the first and second elastomeric membranes 21,22.

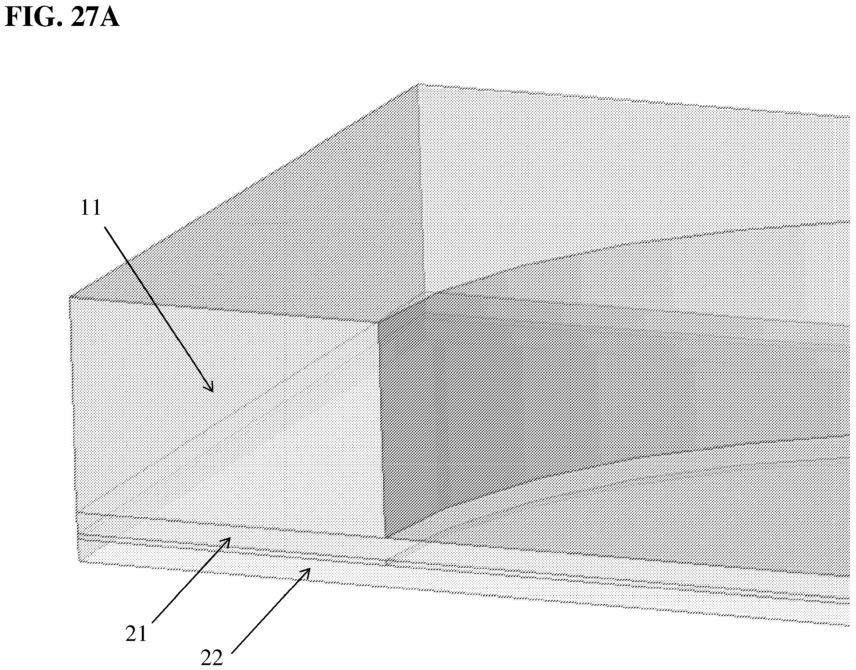

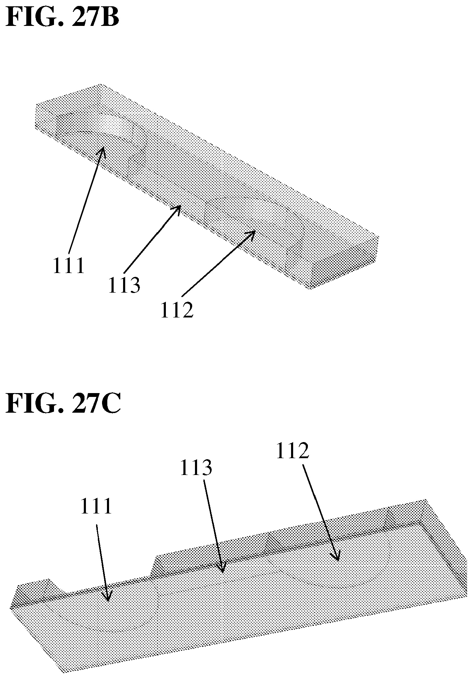

**FIGS. 27A-27C** show a cross-section of an embodiment of the first elastomeric membrane 21 and the second elastomeric membrane 22 attached to each other. An inlet port 224 and/or a chamber port 225 can be positioned on the first elastomeric membrane 21. The inlet port 224 and the chamber port 225 can be formed of any suitable material, for example cyclic olefin copolymer (COC), and can be provided by a single piece of material or be distinct pieces of material. In an embodiment, the first chassis 11 can provide the inlet port 224 and/or the chamber port 225, preferably as part of the magnetic bead handling mechanism 300, for example in vertical alignment with the first mixing chamber 111 and the second mixing chamber 112 respectively. The pistons 53 can move in the inlet port 224 and/or the chamber port 225 to push against the first elastomeric membrane 21 and/or the second elastomeric membrane 22 to urge the one or more reagents 16 therein to the other mixing chamber or a downstream position.

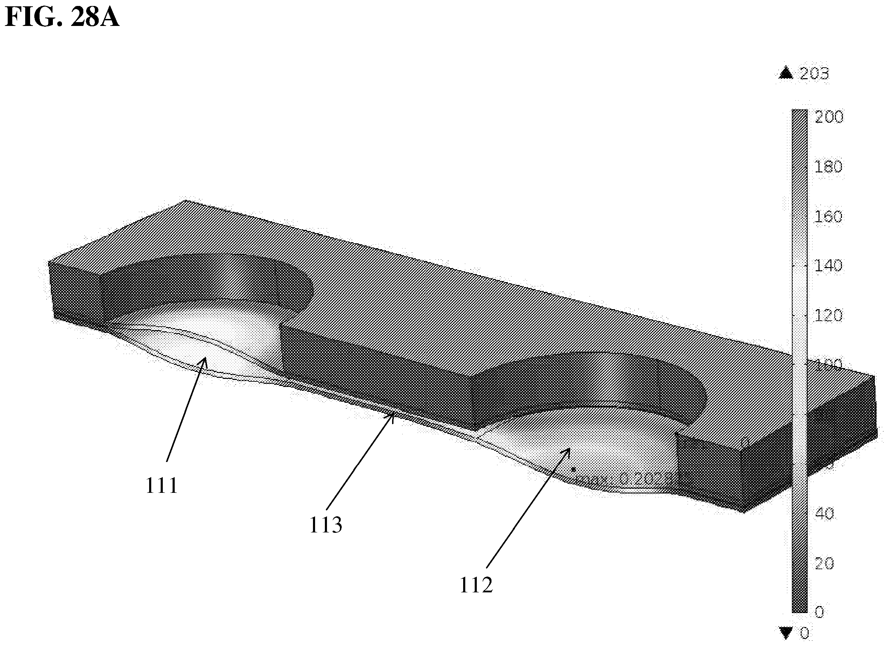

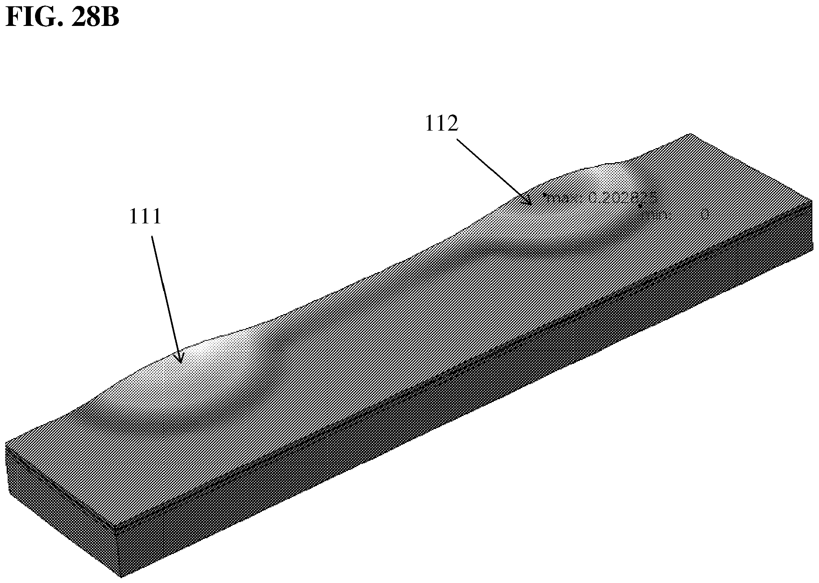

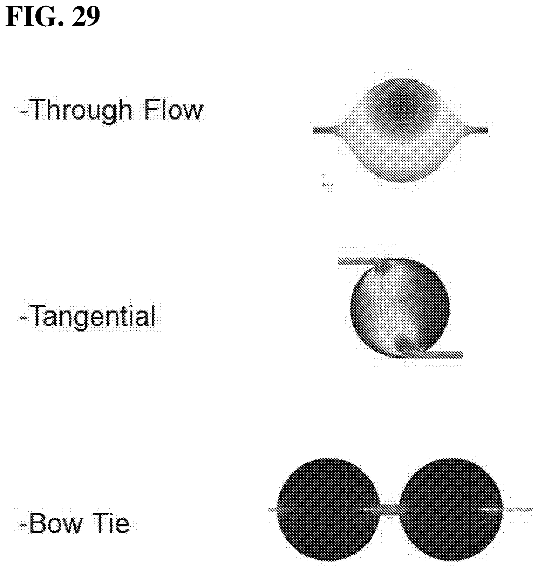

**FIGS. 28A** and **28B** show displacement of the unsealed portions 21b,22b of the first and second elastomeric membranes 21,22 in a cross-section thereof. As shown in these figures, the fluid displaces the elastomeric membranes 21,22 from each other to form a channel and exerts even greater displacement to form a chamber. **FIG. 29** shows the flow distribution in such a "Bow-Tie" configuration relative to other geometries.

The first and second mixing chambers 111,112 can be utilized for one or more steps of a DNA purification process performed in the cartridge 10. For example, magnetic beads can be employed in the first and second mixing chambers 111,112. As used herein in reference to the magnetic beads, "dispersing" refers to re-suspending beads in fluid so they can be transported, and "mixing" refers to re-suspending beads and then performing steps to ensure sufficient bead surface area contacts a particular reagent (sample/wash) for the required timescale. When the magnetic beads are transported, the magnetic beads must reach the correct chamber rather than diffusing or drifting beyond this point. Therefore, in an embodiment, the magnetic beads may be fixedly positioned ("trapped") by one or more of the magnets 301, 302 as a solution of the magnetic beads is injected into the first mixing chamber 111.

The magnetic beads can disperse far into the first and second mixing chambers 111,112 when injected but can be trapped precisely if the magnetic beads are injected while one or more of the magnets 301, 302 are present. In an embodiment, the magnetic beads can be collected in less than one minute, and collection of the magnetic beads can be assisted if the one or more of the magnets 301, 302 are moved relative to the first and second mixing chambers 111,112 rather than maintained in a stationary position.

Flow within the first and second mixing chambers 111,112 can be generated by an inlet to one of the first and second mixing chambers 111,112 and/or by pumping fluid by depressing and releasing one or more of the first and second elastomeric membranes 21,22.

A round shape of the first and second mixing chambers 111,112 can result in stagnant areas at the edges thereof, where velocity is low. Magnetic beads positioned in these areas may be difficult to dislodge. Therefore, in a preferred embodiment, the one or more mixing channels 113 can be narrow, for example having a width of 1-2 mm, and a length of 3-5 mm, to concentrate fluid flow and ensure a high velocity therein. Magnetic beads can be collected in the one or more mixing channels 113 and readily dispersed when the magnets 301, 302 are removed.

The magnetic beads can be substantially uniformly dispersed and/or mixed by spreading the magnetic beads with a moving magnet and/or using oscillating flow. The oscillating flow can be generated by depressing and releasing one or more of the first and second elastomeric membranes 21,22 (e.g., using the pistons 53) and/or by directing flow using a syringe connected to an inlet or an outlet of the first and second mixing chambers 111,112.

As a non-limiting example, the Sanger method may be used in the cartridge 10. In this embodiment, magnetic beads can be released in solution, the magnetic beads can be transferred to the first mixing chamber 111, the sample can be transferred into the first mixing chamber 111, the magnetic beads can be re-suspended if needed and/or desired, the magnetic beads can be fixedly positioned in the first mixing chamber 111 by one or more magnets while subjected to a first wash, the magnetic beads can be re-suspended if needed and/or desired, the magnetic beads can be fixedly positioned in the first mixing chamber 111 by one or more of the magnets 301, 302 while subjected to a second wash, the magnetic beads can be re-suspended if needed and/or desired, elution buffer can be added to the first mixing chamber 111, the magnetic beads can be re-suspended if needed and/or desired, and the elution buffer can be transferred from the first mixing chamber 111 to the second mixing chamber 112. In this non-limiting example, these steps are preferably performed in the order they are listed, but one or more of the steps may be omitted, and additional steps may be added.

In an embodiment, the magnets 301, 302 can trap the magnetic beads in the first and second mixing chambers 111,112 during ethanol washes and can trap the magnetic beads in the one or more mixing channels 113 prior to elution steps with elution buffer. In an embodiment, the magnets 301, 302 can be positioned within the pistons 53 and can be retractable within the pistons 53. This configuration of the magnets 301, 302 can trap the magnetic beads rapidly inside the first and second mixing chambers 111,112. After the magnetic beads are collected by the magnets 301, 302, the collected magnetic beads can be drawn into the one or more mixing channels 113.

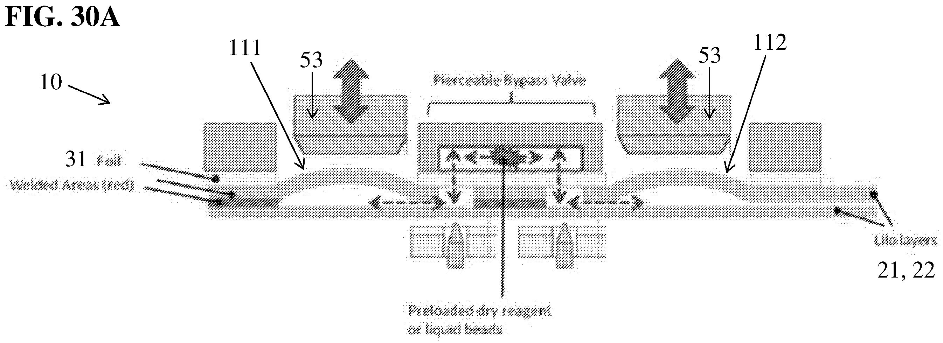

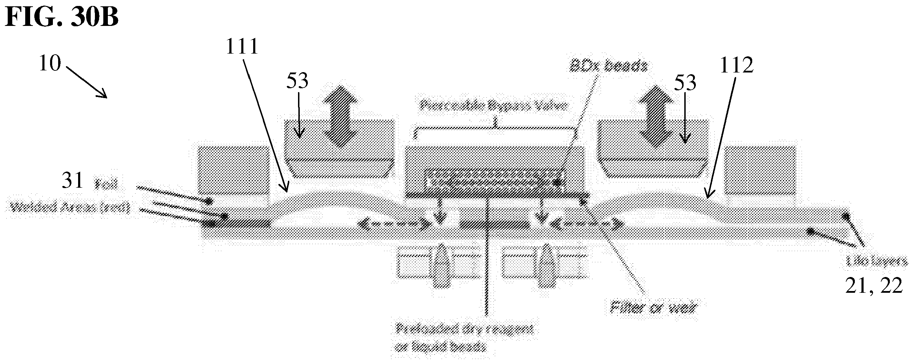

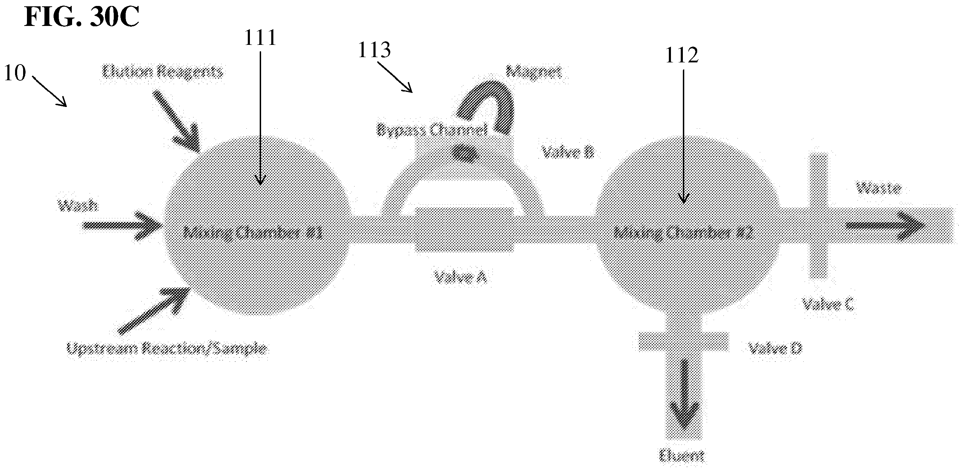

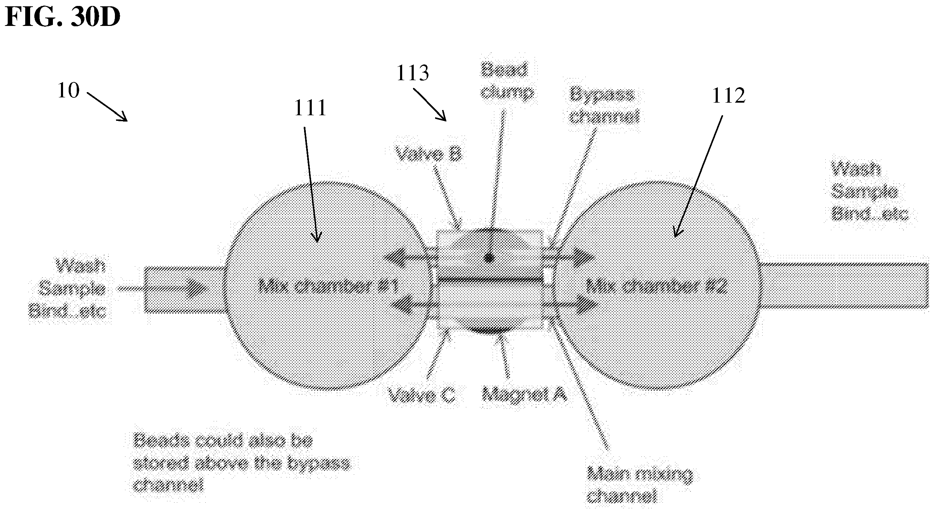

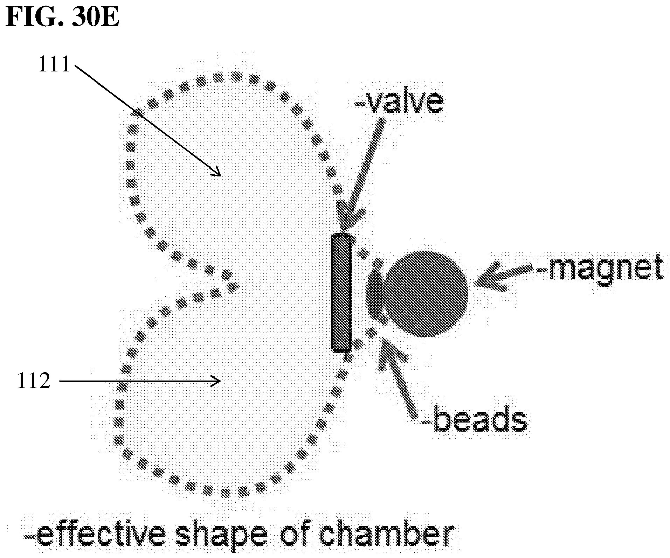

**FIGS. 30A-30E** generally illustrate various configurations that can be employed in the magnetic bead handling mechanism 300. As shown in **FIGS. 30A-30D****,** the mixing channel 113 can comprise a pierceable bypass valve which can contain pre-loaded dry reagent or liquid beads. As shown in **FIG. 30E****,** not forming part of the present invention, the first and second mixing chambers 111,112 can form a wide "kidney-shaped" chamber, and the mixing channel 113 can be absent. The magnetic beads can be trapped in a valved-off region at an end of the chambers 111,112.

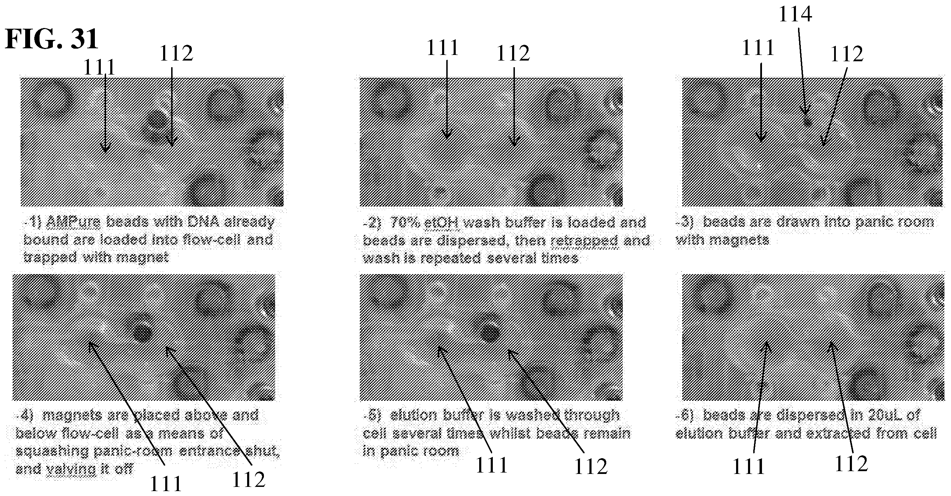

In some embodiments, an additional chamber 114 can be connected to one or both of the first and second mixing chambers 111,112. A valve can selectively block the connection to the additional chamber 114 to prevent the contents of the additional chamber 114 from exiting the chamber 114, such as during washing of the corresponding one of the first and second mixing chambers 111,112. A non-limiting example of an experiment utilizing the additional chamber 114 is shown in **FIG. 31****.**

### Elastomeric Membrane Physical Properties

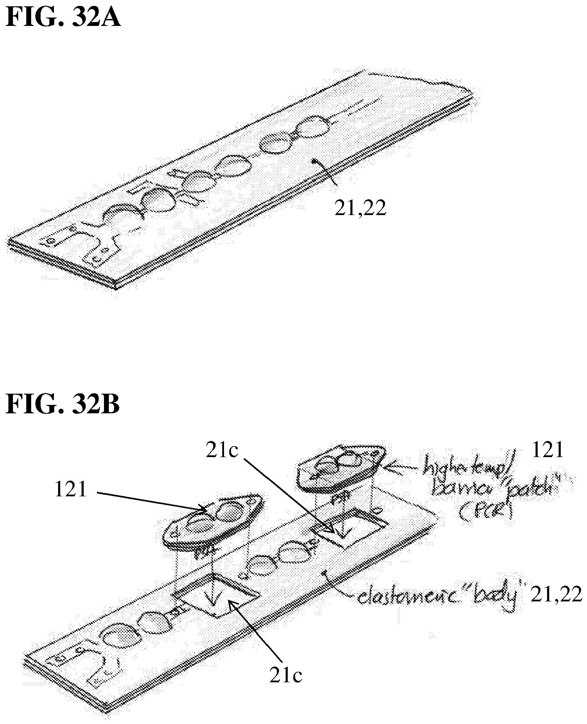

Preferably the material of the first and second elastomeric membranes 21,22 has one or more of the following characteristics: capable of piercing foil by being pushed against the foil be a plunger, as discussed previously herein; capable of being inflated to form a chamber, as discussed previously herein; formable; weldable; biologically compatible; capable of maintaining integrity at a 100 °C operating temperature; and/or performing as a high temperature vapor barrier. For example, some thermoplastic elastomers such as polyvinyl chloride are capable of continued functionality at higher temperatures, and some high barrier films such as a co-extrusion of polyethylene and polyurethane can be employed. Therefore, in a preferred embodiment generally illustrated in **FIG. 32A****,** the first elastomeric membrane 21 is formed from a single piece of material, and the second elastomeric membrane 22 is formed from a single piece of material.

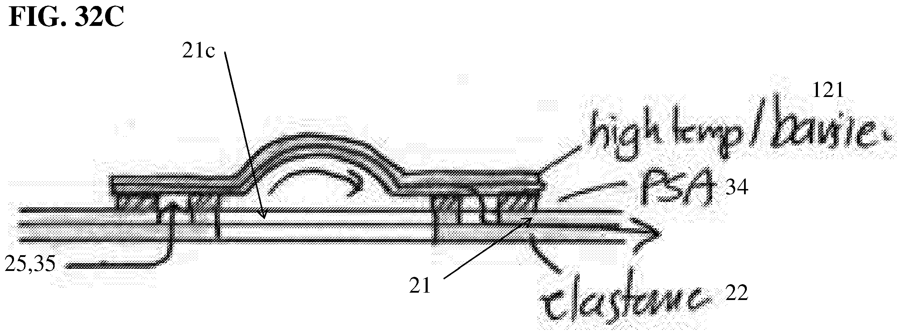

As generally illustrated in **FIGS. 32B** and **32C****,** an embodiment of the cartridge 10 uses one or more first elastomeric patches 121 on the first elastomeric membrane 21. The first elastomeric membrane 21 can comprise open sections 21c with which the one or more first elastomeric patches 121 can be substantially co-extensive such that the combination of the one or more first elastomeric patches 121 and the first elastomeric membrane 21 form a continuous layer. The one or more first elastomeric patches 121 are preferably a different material than the first elastomeric membrane 21, for example a material with higher temperature tolerance and/or greater vapor barrier characteristics relative to the material of the first elastomeric membrane 21. The one or more first elastomeric patches 121 can be used in areas of the cartridge 10 where the fluid traversing the cartridge 10 is subjected to higher temperatures. The cartridge 10 can comprise one or more second elastomeric patches (not shown) which can cooperate with the second elastomeric membrane 22 similarly to the one or more first elastomeric patches 121 and the first elastomeric membrane 21 as discussed above.

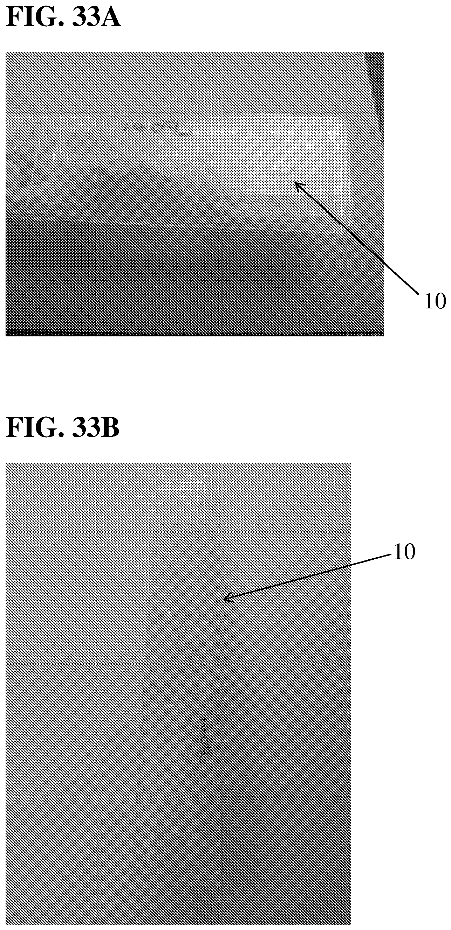

As a non-limiting example, **FIGS. 33A** and **33B** show an embodiment of the cartridge 10 in which an elastomer is bonded directly to a moulded lane, for example a lane moulded into polypropylene. The elastomer can be directly bonded by heat-sealing, a pressure-sensitive adhesive, or any other suitable means known to the skilled artisan. Additionally or alternatively to the direct bonding, the elastomer can be bonded to a foil layer that is on the moulded lane, and the foil layer can form the lids of the chambers, optionally with a pressure sensitive adhesive layer. An externally applied feature can be used to evacuate fluid from the channels.

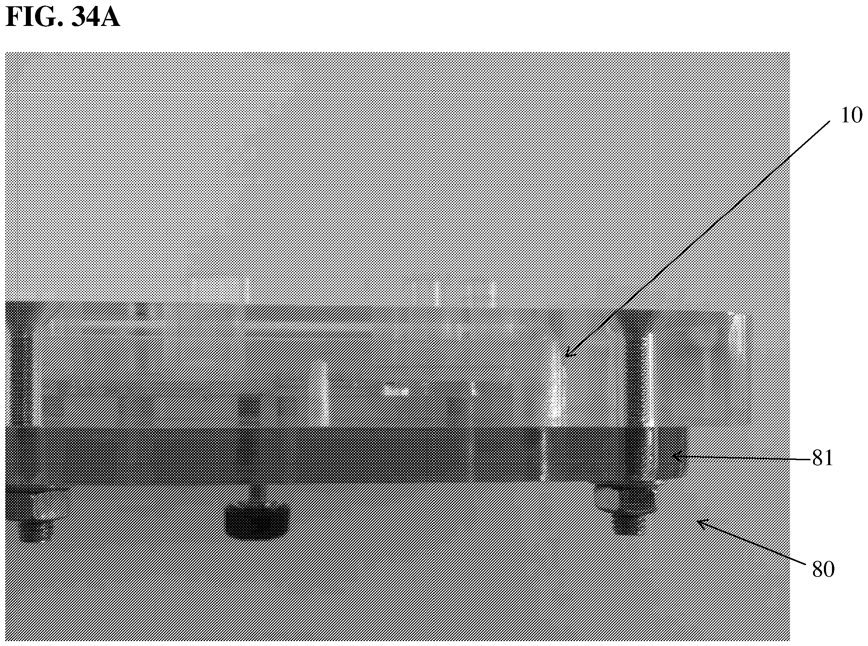

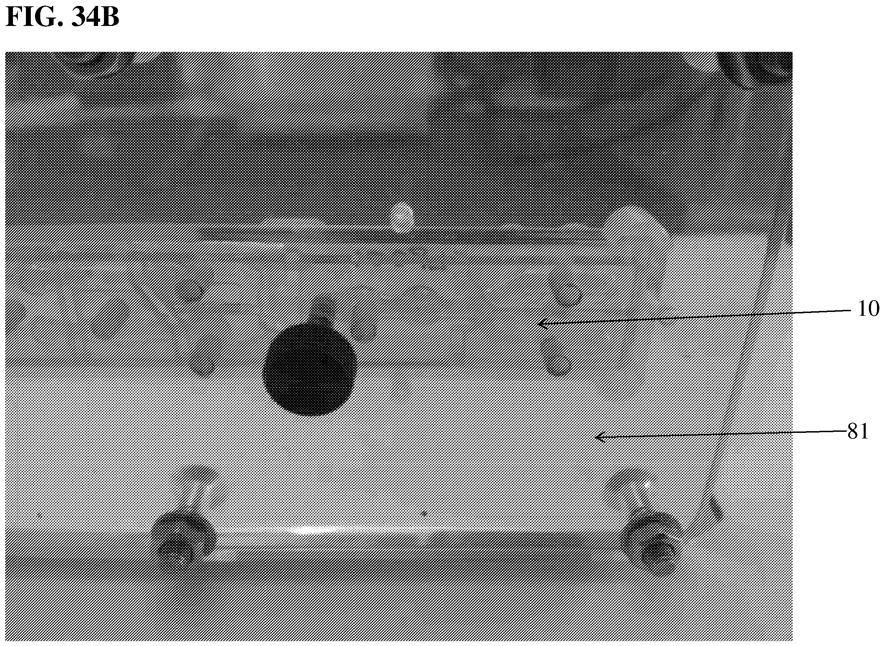

**FIGS. 34A** and **34B** show this embodiment of the cartridge 10 clamped into a fixture 80 that can comprise a clamping plate 81, with a valve comprising a screw that can rotated one direction to move the valve to the open position and can be rotated an opposite direction to move the valve to the closed position. Preferably, the clamping plate 81 comprises recesses configured to allow the unsealed portions 21b,22b of the first and second elastomeric membranes 21,22 to inflate when not closed by the valve and when reached by the one or more reagents 16. The clamping plate 81 can prevent the layers in the cartridge 10, such as the first and second elastomeric membranes 21,22, from delaminating.

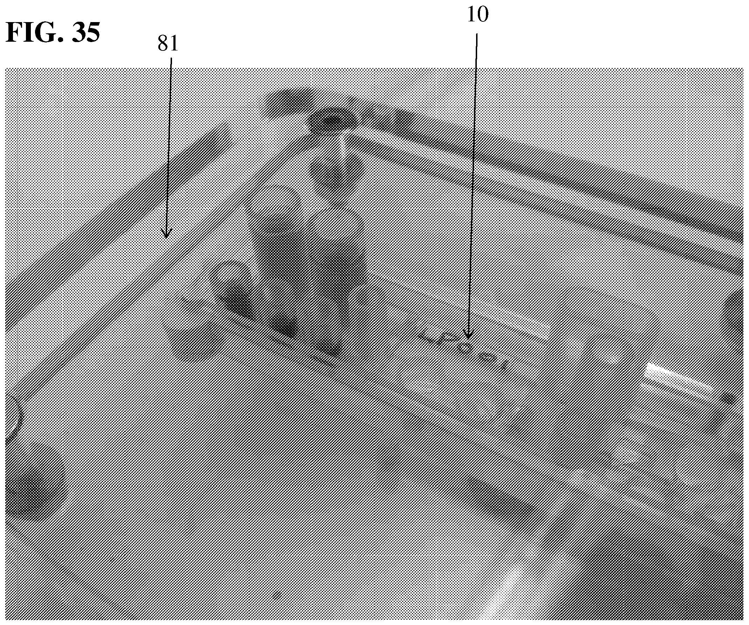

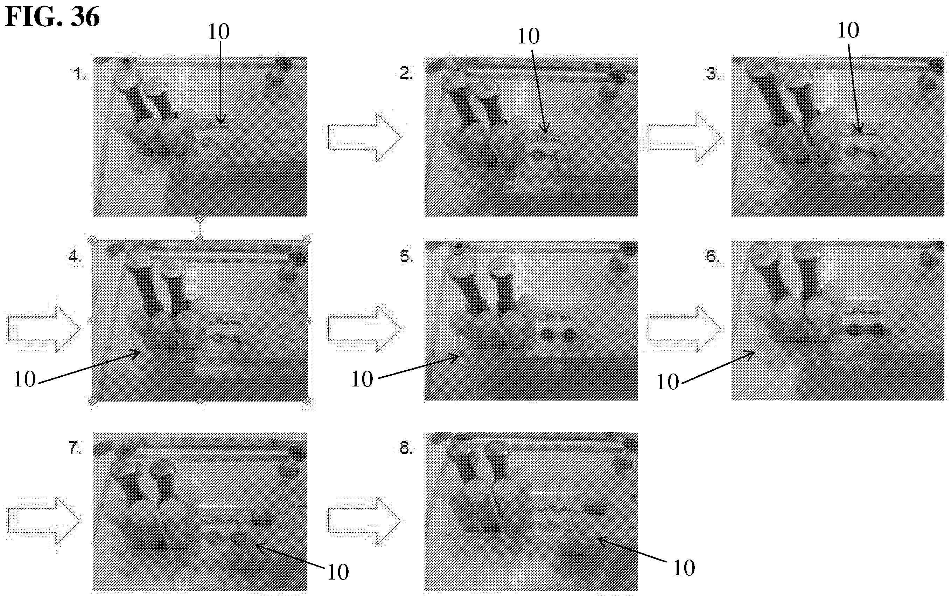

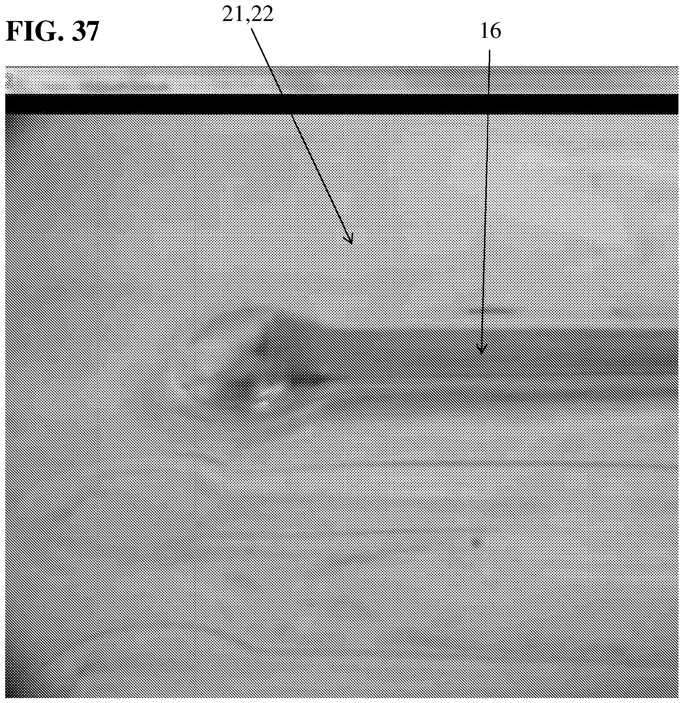

**FIG. 35** shows fluid loading into this embodiment of the cartridge 10. **FIG. 36** shows progression of fluid through the cartridge 10, for example fluid dosing and mixing. **FIG. 37** shows the one or more reagents 16 flowing between the unsealed portions 21b,22b of the first and second membranes 21,22, thereby forming a channel in the cartridge 10.

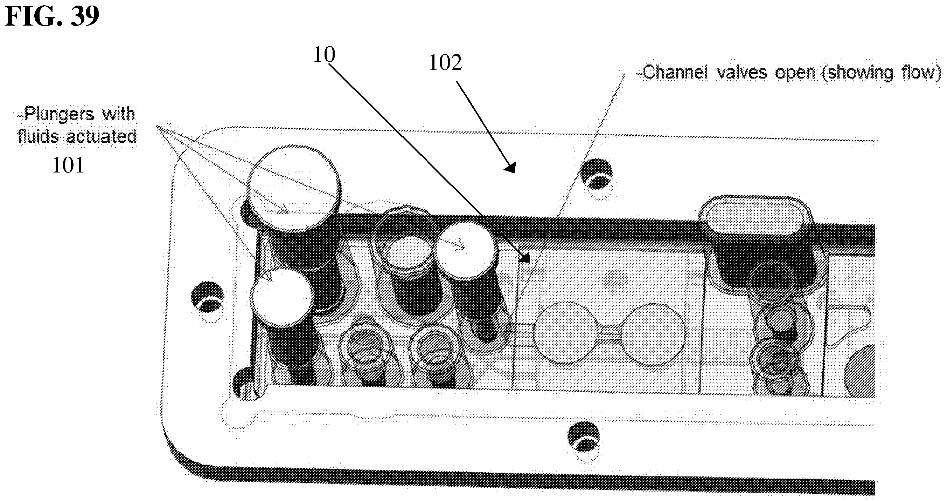

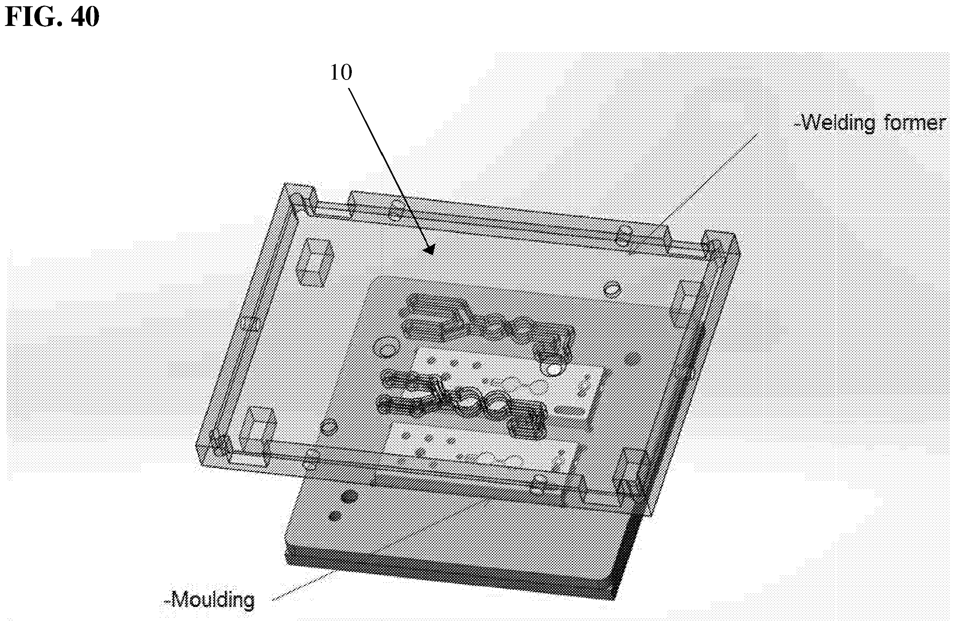

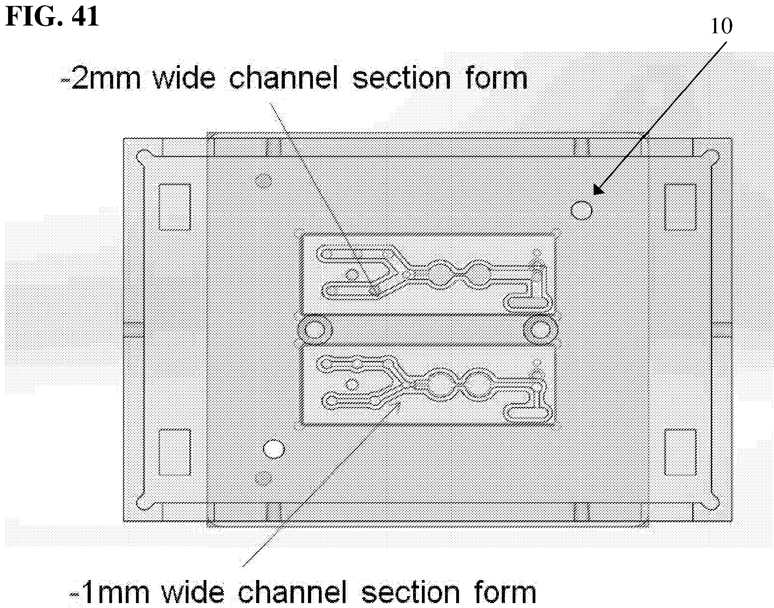

**FIGS. 38-41** show an embodiment of a system 100 comprising the cartridge 10. The system 100 can comprise plungers 101 that can be used to dose the one or more reagents 16 and/or other materials into the cartridge 10, a clamping frame 102 to which the cartridge 10 can reversibly connect, an intermediate piece 104 to which the clamping frame 102 can reversibly connect, and a base 105 to which the intermediate piece 103 can reversibly connect. The base 105 can comprise one or more legs 106 configured to position the cartridge 10 at an elevated location.

In an embodiment, the base 106 can comprise toggle clamps 107 that are configured to be separately operated to thereby actuate the channels in the cartridge 10, for example by moving between a closed and an open position. Moving one of the toggle clamps 106 into a closed position can urge the one or more reagents 16 positioned in the corresponding section of the cartridge 10 to a downstream location in the cartridge 10. For example, each of the toggle clamps 107 can comprise a section of the support block 12 and a section of the compliant layer 20 affixed on the section of the support block 12.

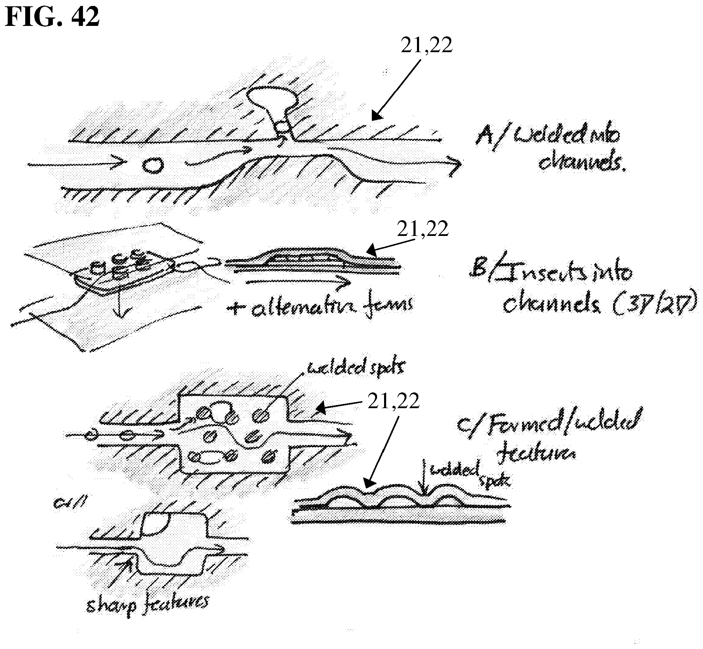

**FIG. 42** generally illustrates an embodiment of the cartridge 10 in which bubble traps are used, preferably between the first and second elastomeric membranes 21,22. The bubble traps can lead to dead volume, thus the bubble traps can be configured to balance the dead volume created and the effectiveness at scavenging bubbles. **FIG. 42** shows that inserts, for example stereolithography inserts, can be employed as bubble traps. Alternative geometries and materials can be used for the inserts. In an embodiment, the inserts can be formed or fabricated plastic foils.

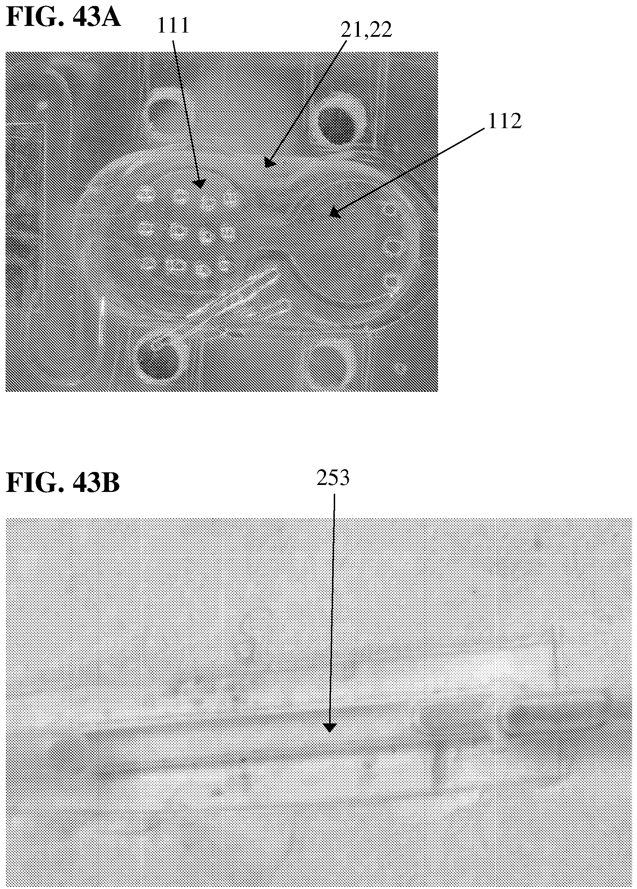

**FIG. 42** contains schematic drawings showing that the welding techniques used to laminate the first and second elastomeric membranes 21,22 can form the bubble traps; for example, sections of the first elastomeric membrane 21 welded to sections of the second elastomeric membrane 22 can form the bubble traps. **FIG. 43A** is a photograph of an embodiment in which sections of the first elastomeric membrane 21 are welded to sections of the second elastomeric membrane 22 can form the bubble traps.

**FIG. 43B** is a photograph of a de-bubbler channel 253. In various embodiments, the de-bubbler channel 253 includes a fluid channel where one wall is a porous membrane. As a fluid containing air bubbles is pushed along the channel under pressure, the air is vented through the membrane and is thereby removed from the fluid.

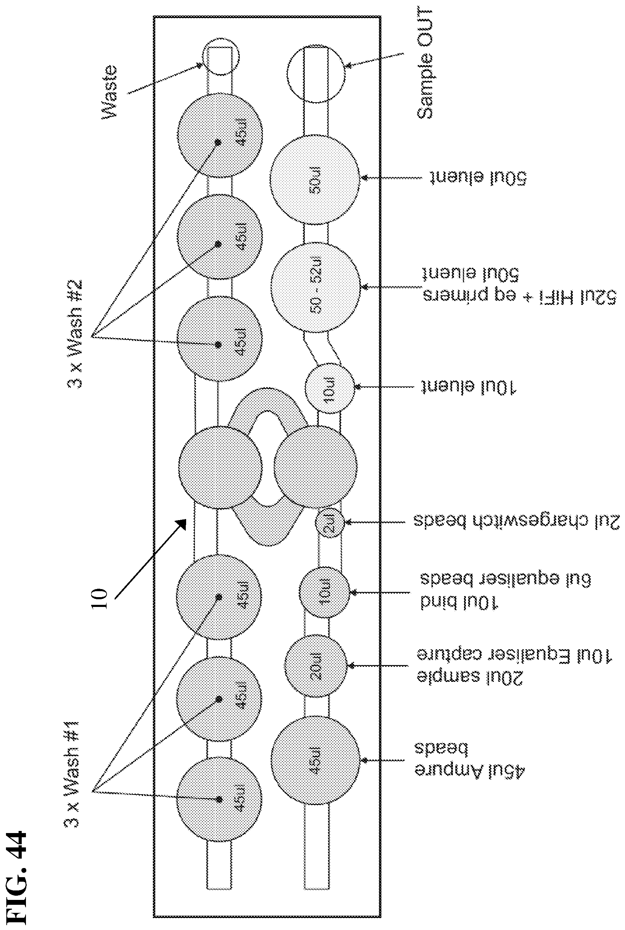

**FIG. 44** is a schematic diagram of a non-limiting example of reagents and their positioning in the cartridge 10 in an embodiment of the cartridge 10.

Various changes and modifications to the presently preferred embodiments described herein will be apparent to those skilled in the art. Such changes and modifications can be made without departing from the scope of the present subject matter and without diminishing its intended advantages. Therefore, such changes and modifications are covered by the appended claims.

## Claims

1. An automated system for the thermal processing of reagents, the system comprising:
a heater block (52) that emits heat;
a first elastomeric membrane (21); and
a second elastomeric membrane (22) positioned adjacent the first elastomeric membrane, wherein a portion of the second elastomeric membrane is sealed to a portion of the first elastomeric membrane to define a first mixing chamber (111) positioned and arranged to receive the heat from the heater block, wherein the first and second elastomeric membranes define a second mixing chamber (112) positioned and arranged to receive the heat from the heater block, and comprising a first piston (53) and a second piston (53), opposite the heater block, and wherein a compliant layer (20) is positioned under the first elastomeric membrane (21),a portion of the complaint layer reversibly closes a section of a channel formed by the first and the second elastomeric membrane upstream from the first and second mixing chambers and another portion of the compliant layer reversibly closes a section of the channel downstream from the first and second mixing chambers,
**characterized in that** the automated system is configured such that, in operation, the first piston is adapted to reversibly depress the first mixing chamber to urge one or more reagents (16) from the first mixing chamber to the second mixing chamber, and such that, in operation, the second piston is adapted to reversibly depress the second mixing chamber to urge the one or more reagents from the second mixing chamber to the first mixing chamber, and **in that** the first mixing chamber and the second mixing chamber are connected to each other by one or more mixing channels (113) that have a width smaller than the width of the channel upstream from the first mixing chamber and/or the width of a channel downstream from the second mixing chamber.

2. The system of claim 1, wherein the piston comprises a magnet (301).

3. The system of claim 1, wherein a cartridge comprises the first and second elastomeric membranes, and an instrument configured to interact with the cartridge comprises the heater block.

4. A method comprising the steps of:
providing a system of claim 1,
introducing fluid into a channel, the channel defined by a portion of a first elastomeric membrane (21) sealed to a portion of a second elastomeric membrane (22), wherein the first and second elastomeric membranes comprise sealed portions (31a) circumscribing the unsealed portions (31b),
wherein prior to introduction of fluid into the channel the unsealed portion of the first elastomeric membrane substantially abuts the unsealed portion of the second elastomeric membrane, and the introduction of the fluid forces separation of the unsealed portion of the first elastomeric membrane from the unsealed portion of the second elastomeric membrane.

5. The method of claim 4, comprising applying pressure to a first unsealed portion so that the fluid flows through the channel into a second unsealed portion, wherein the first unsealed portion returns to substantial abutment with an opposite unsealed portion.

6. The method of claim 4, comprising storing the fluid in a pot, the pot defining an upper passage through which the fluid is introduced into the channel.

## Patentansprüche

1. Automatisiertes System für die thermische Verarbeitung von Reagenzien, das System umfassend:
einen Heizblock (52), der Hitze abgibt;
eine erste Elastomermembran (21); und
eine zweite Elastomermembran (22), die angrenzend an die erste Elastomermembran positioniert ist, wobei ein Abschnitt der zweiten Elastomermembran an einem Abschnitt der ersten Elastomermembran abgedichtet ist, um eine erste Mischkammer (111) zu definieren, die positioniert und angeordnet ist, um die Hitze von dem Heizblock aufzunehmen, wobei die erste und die zweite Elastomermembran eine zweite Mischkammer (112) definieren, die positioniert und angeordnet ist, um die Hitze von dem Heizblock aufzunehmen, und umfassend einen ersten Kolben (53) und einen zweiten Kolben (53) gegenüber des Heizblocks, und wobei eine nachgiebige Schicht (20) unter der ersten Elastomermembran (21) positioniert ist, ein Abschnitt der nachgiebigen Schicht einen Bereich eines Kanals reversibel verschließt, der durch die erste und die zweite Elastomermembran stromaufwärts von der ersten und der zweiten Mischkammer ausgebildet wird, und ein anderer Abschnitt der nachgiebigen Schicht einen Bereich des Kanals stromabwärts von der ersten und der zweiten Mischkammer reversibel verschließt,
**dadurch gekennzeichnet, dass** das automatisierte System derart konfiguriert ist, dass der erste Kolben in Betrieb angepasst ist, um die erste Mischkammer reversibel niederzudrücken, um ein oder mehrere Reagenzien (16) von der ersten Mischkammer zu der zweiten Mischkammer zu drängen, und derart, dass der zweite Kolben in Betrieb angepasst ist, um die zweite Mischkammer reversibel niederzudrücken, um das eine oder die mehreren Reagenzien von der zweiten Mischkammer zu der ersten Mischkammer zu drücken, und dadurch, dass die erste Mischkammer und die zweite Mischkammer durch einen oder mehrere Mischkanäle (113) miteinander verbunden sind, die eine Breite aufweisen, die kleiner als die Breite des Kanals stromaufwärts von der ersten Mischkammer ist, und/oder die Breite eines Kanals stromabwärts der zweiten Mischkammer ist.

2. System nach Anspruch 1, wobei der Kolben einen Magneten (301) umfasst.

3. System nach Anspruch 1, wobei eine Kartusche die erste und die zweite Elastomermembran umfasst und ein Instrument, das konfiguriert ist, um mit der Kartusche zusammenzuwirken, den Heizblock umfasst.

4. Verfahren, umfassend die Schritte:
Bereitstellen eines Systems nach Anspruch 1,
Einführen von Fluid in einen Kanal, wobei der Kanal durch einen Abschnitt einer ersten Elastomermembran (21) definiert ist, der an einem Abschnitt einer zweiten Elastomermembran (22) abgedichtet ist, wobei die erste und die zweite Elastomermembran abgedichtete Abschnitte (31a) umfassen, die die nicht abgedichteten Abschnitte (31b) umgeben,
wobei vor dem Einführen von Fluid in den Kanal der nicht abgedichtete Abschnitt der ersten Elastomermembran im Wesentlichen an den nicht abgedichteten Abschnitt der zweiten Elastomermembran anliegt und das Einführen des Fluids eine Trennung des nicht abgedichteten Abschnitts der ersten Elastomermembran von dem nicht abgedichteten Abschnitt der zweiten Elastomermembran erzwingt.

5. Verfahren nach Anspruch 4, umfassend ein Ausüben von Druck auf einen ersten nicht abgedichteten Abschnitt, sodass das Fluid durch den Kanal in einen zweiten nicht abgedichteten Abschnitt strömt, wobei der erste nicht abgedichtete Abschnitt zu einer wesentlichen Anlage mit einem gegenüberliegenden nicht abgedichteten Abschnitt zurückkehrt.

6. Verfahren nach Anspruch 4, umfassend ein Aufbewahren des Fluids in einem Topf, wobei der Topf einen oberen Durchgang definiert, durch den das Fluid in den Kanal eingeführt wird.

## Revendications

1. Système automatisé pour le traitement thermique de réactifs, le système comprenant :
un bloc de chauffage (52) qui émet de la chaleur ;
une première membrane élastomère (21) ; et
une seconde membrane élastomère (22) positionnée adjacente à la première membrane élastomère, dans lequel une partie de la seconde membrane élastomère est scellée à une partie de la première membrane élastomère pour définir une première chambre de mélange (111) positionnée et agencée pour recevoir la chaleur provenant du bloc de chauffage, dans lequel les première et seconde membranes élastomères définissent une seconde chambre de mélange (112) positionnée et agencée pour recevoir la chaleur provenant du bloc de chauffage, et comprenant un premier piston (53) et un second piston (53), opposés au bloc de chauffage, et dans lequel une couche souple (20) est positionnée sous la première membrane élastomère (21), une partie de la couche souple ferme de manière réversible une section d'un canal formé par la première et la seconde membrane élastomère en amont des première et seconde chambres de mélange et une autre partie de la couche souple ferme de manière réversible une section du canal en aval des première et seconde chambres de mélange,
**caractérisé en ce que** le système automatisé est configuré de telle sorte que, en fonctionnement, le premier piston est conçu pour appuyer de manière réversible sur la première chambre de mélange pour presser un ou plusieurs réactifs (16) depuis la première chambre de mélange vers la seconde chambre de mélange, et de telle sorte que, en fonctionnement, le second piston est conçu pour appuyer de manière réversible sur la seconde chambre de mélange pour presser le ou les réactifs depuis la seconde chambre de mélange vers la première chambre de mélange, et **en ce que** la première chambre de mélange et la seconde chambre de mélange sont raccordées l'une à l'autre par un ou plusieurs canaux de mélange (113) qui ont une largeur plus petite que la largeur du canal en amont de la première chambre de mélange et/ou la largeur d'un canal en aval de la seconde chambre de mélange.

2. Système selon la revendication 1, dans lequel le piston comprend un aimant (301).

3. Système selon la revendication 1, dans lequel une cartouche comprend les première et seconde membranes élastomères, et un instrument configuré pour interagir avec la cartouche comprend le bloc de chauffage.

4. Procédé comprenant les étapes consistant à :
fournir un système selon la revendication 1,
introduire un fluide dans un canal, le canal étant défini par une partie d'une première membrane élastomère (21) scellée à une partie d'une seconde membrane élastomère (22), dans lequel les première et seconde membranes élastomères comprennent des parties scellées (31a) entourant les parties non scellées (31b),
dans lequel avant introduction de fluide dans le canal la partie non scellée de la première membrane élastomère vient sensiblement en butée contre la partie non scellée de la seconde membrane élastomère, et l'introduction du fluide force une séparation de la partie non scellée de la première membrane élastomère par rapport à la partie non scellée de la seconde membrane élastomère.

5. Procédé selon la revendication 4, comprenant l'application de pression à une première partie non scellée de sorte que le fluide s'écoule à travers le canal dans une seconde partie non scellée, dans lequel la première partie non scellée revient sensiblement en butée avec une partie non scellée opposée.

6. Procédé selon la revendication 4, comprenant le stockage du fluide dans un récipient, le récipient définissant un passage supérieur à travers lequel le fluide est introduit dans le canal.
